(19) Europäisches Patentamt
European Patent Office
Office européen des brevets

(11) Publication number: **0 301 850 A2**

(12) # EUROPEAN PATENT APPLICATION

(21) Application number: 88306947.8

(22) Date of filing: 28.07.88

(51) Int. Cl.$^4$: **C 07 K 7/20**
A 61 K 37/02, A 61 K 31/56

(30) Priority: 31.07.87 US 80518

(43) Date of publication of application: 01.02.89 Bulletin 89/05

(84) Designated Contracting States: AT BE CH DE ES FR GB GR IT LI LU NL SE

(71) Applicant: SYNTEX (U.S.A.) INC.
3401 Hillview Avenue P.O. Box 10850
Palo Alto California 94303 (US)

(72) Inventor: Vickery, Brain H.
20279 Carol Lane
Saratoga California 95070 (US)

(74) Representative: Armitage, Ian Michael et al
MEWBURN ELLIS & CO. 2/3 Cursitor Street
London EC4A 1BQ (GB)

(54) **LHRH antagonist analogs and 19-nor-progestational steroids for therapy.**

(57) A combination of an LHRH antagonist and a progestational agent is disclosed, for treating female gynecological disorders based on gonadal steroid production.

EP 0 301 850 A2

## Description

# LHRH ANTAGONIST ANALOGS AND 19-NOR-PROGESTATIONAL STEROIDS FOR THERAPY

## BACKGROUND OF THE INVENTION

Luteinizing hormone (abbreviated LH) and follicle stimulating hormone (FSH) are released from the anterior pituitary gland under the control of luteinizing hormone releasing hormone (abbreviated LHRH, GnRH, or LH/FSH-RH) produced in the hypothalamic region. LH and FSH act on the gonads to stimulate the synthesis of steroid hormones and to stimulate gamete maturation. The pulsatile release of LHRH, and thereby the release of LH and FSH, controls the reproductive cycle in animals and humans.

LHRH also affects the placenta, and therefore the gonads indirectly, by causing the synthesis and release of chorionic gonadotropin (CG).

The natural releasing hormone LHRH is a decapeptide comprised of naturally occurring amino acids (which have the L-configuration except for the achiral amino acid glycine). Its sequence is as follows:

```
(pyro) Glu-His-Trp-Ser-Tyr-Gly-Leu-Arg-Pro-Gly-NH2
        1   2   3   4   5   6   7   8   9   10
```

Analogs of this natural material are often described in abbreviated form by showing the nature of the substituent of a given amino acid, superscribed by its location, followed by "LHRH."

Many analogs of LHRH have been studied, and a very large majority are of insufficient biological activity to be clinically useful. However, certain select modifications have a potentiating effect on agonist biological activity. A significant enhancement to agonist activity is obtained by changing the 6-position residue from Gly to a D-amino acid.

In addition to agonists, analogs have been prepared which are competitive antagonists to LHRH, all of which require deletion or replacement of the histidyl residue at position 2: Vale, W., et al., Science, 176: 933 (1972). In general, it appears that a D-amino acid placed in the sequence at that position gives the best activity: Rees, R. W. A., et al., J. Med. Chem. 17: 1016 (1974).

LHRH antagonist analogs are such as, but not limited to, those described in U.S. Pat. No. 4,690,916 and copending European Patent Application No. 88300927.6 (Canadian Patent Application S.N. 558099, Australian Patent Application No. 11265, New Zealand Patent Application No. 223403). The compounds of the copending European patent application are those of the formula:

```
A-B-C-Ser-D-E-F-G-Pro-J        (I)
1 2 3  4  5 6 7 8  9  10
```

or a pharmaceutically acceptable salt thereof, wherein

A is an amino acyl residue selected from the group consisting of either the D- or the L- isomer of: N-Ac-D,L-$\Delta^{3,4}$-prolyl, N-Ac-D,L-prolyl, N-Ac-D,L-Phenylalanyl, N-Ac-D,L-p-chlorophenylalanyl, N-Ac-D,L,-p-fluorophenylalanyl, N-Ac-3-(1-naphthyl)-D,L-alanyl, N-Ac-3-(2-naphthyl)-D,L-alanyl, and N-Ac-3-(2,4,6-trimethylphenyl)-D,L-alanyl;

B is an amino acyl residue selected from the group consisting of D-phenylalanyl, D-p-chlorophenylalanyl, D-p-fluorophenylalanyl, D-p-nitrophenylalanyl, 2,2-diphenylglycyl, D-α-methyl-p-chlorophenylalanyl and 3-(2-naphthyl)-D-alanyl;

C is an amino acyl residue selected from the group consisting of D-tryptophanyl, D-phenylalanyl, 3-(3-pyridyl)-D-alanyl, and 3-(2-naphthyl)-D-alanyl;

D is an amino acyl residue selected from the group consisting of L-phenylalanyl, L-tyrosyl, and 3-(3-pyridyl)-alanyl, arginyl, or G;

E is 3-(2-naphthyl)-D-alanyl, 3-(3-pyridyl)-D-alanyl, D-tyrosyl, D-tryptophanyl, D-nicotinyl-lysyl, pyridylacetyl-lysyl, D-Glu(AA) or G;

F is an amino acyl residue selected from the group consisting of L-leucyl, L-norleucyl, L-phenylalanyl, L-tryptophanyl, and 3-(2-naphthyl)-L-alanyl;

G is an amino acyl residue selected from the group consisting of the radicals represented by the following structural formulas:

(a)

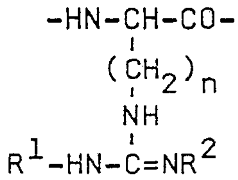

(II)

wherein
n is 1 to 5;
$R^1$ is alkyl of 1 to 6 carbon atoms or fluoroalkyl;
$R^2$ is hydrogen or $R^1$; or $R^1$-HN-C=$NR^2$ is a ring represented by the following structural formulas:

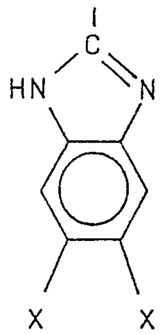

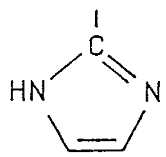

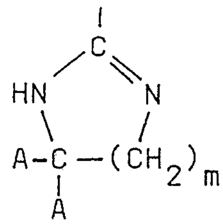

wherein m is 1 to 4; A is hydrogen or alkyl of 1 to 6 carbon atoms; and X is halo or A; and

(b)

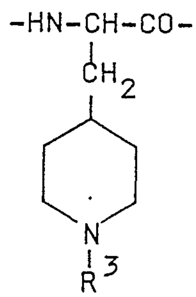

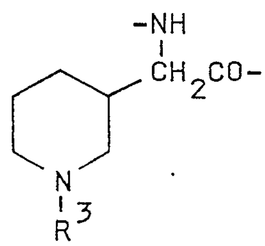

wherein $R^3$ is hydrogen, alkyl of 1 to 6 carbon atoms, phenyl or phenylloweralkyl; and
J is D-alaninamide; D-leucinamide; glycinamide; or $-NHR^4$ wherein $R^4$ is lower alkyl or $NHCONH_2$, and can be prepared by methods known in the art.

Such compounds are extremely effective in shutting down gonadal function in mammals, and thus are useful therapeutic agents for a range of syndromes and diseases which are dependent on or aggravated by gonadal steroid production. The LHRH antagonists described patent and in said patent application are particularly advantageous because they are potent anti-ovulatory agents, and yet unlike other antagonists, they have minimal histamine-releasing activity. (See, Vickery, Pharmacology of LHRH Antagonists, Furr and Wakeling, Eds., Pharmacology and Clinical Uses of Inhibitors of Hormonal Secretion and Action, Bailliere Tindall, London, 1987, p. 385; Vickery and Nestor, LHRH Analogues: Development and Mechanism of Action, Sem. Reprod. Endocrinology, August 1987). In women, such LHRH antagonists have particular utility for the treatment of endometriosis, breast cancer and uterine tumors such as leiomyomata. However, the withdrawal of ovarian steroids, particularly estrogens, can lead to menopause-like symptoms including vasomotor instability ("hot flashes") and decrease in bone density ("osteoporosis").

Progestational steroids have been used in peri- and post-menopausal women to alleviate hot flashes (Appleby, Norethisterone in the Control of Menopausal Symptoms, Lancet 1, 407, 1962) and also prevent loss of bone mass (Abdalla et al., Prevention of Bone Mineral Loss in Postmenopausal Women by Norethisterone,

Obstet. Gynecol. 66, 789, 1985).

## SUMMARY OF THE INVENTION

According to a first aspect, the present invention is directed to a pharmaceutical composition, comprising a therapeutically effective amount of an LHRH antagonist and a menopausal-symptom-alleviating amount of a 19-nor progestational agent.

According to a second aspect, the invention relates to a pharmaceutical composition comprising a therapeutically effective amount of an LHRH antagonist and a menopausal-symptom-alleviating amount of a 19-nor progestational agent, wherein the LHRH antagonist and the 19-nor progestational agent are in a single formulation.

According to another aspect, the invention is directed to the use of an LHRH antagonist and a menopausal-symptom-alleviating amount of a 19-nor-progestational agent in combination in a composition or administered concomitantly for treating a disease state that is dependent on gonadal steroid production, which use comprises administration of a therapeutically effective amount of an LHRH antagonist in combination with a menopausal-symptom-alleviating amount of a 19-nor progestational agent.

Another aspect of the invention relates to the use of an LHRH antagonist and a menopausal-symptom-alleviating amount of a 19-nor-progestational agent in combination in a composition or administered concomitantly for treating endometriosis, uterine leiomyomata, breast cancer or polycystic ovarian disease or for preventing ovulation in a female mammalian subject.

An additional aspect of the invention relates to a pharmaceutical composition for use in inhibiting ovulation, or treating endometriosis, breast cancer, uterine leiomyomata, or polycystic ovarian disease in a female mammalian subject, said composition comprising a therapeutically effective amount of an LHRH antagonist in combination with a menopausal-symptom-alleviating amount of a 19-nor progestational agent.

Yet another aspect of the invention relates to a method for preparing a pharmaceutical composition which comprises mixing an LHRH antagonist and a menopausal-symptom-alleviating amount of a 19-nor progestational agent with a pharmaceutically acceptable non-toxic carrier or excipient.

## DETAILED DESCRIPTION OF THE PREFERRED EMBODIMENTS

### Abbreviations and Definitions

For the purposes of this invention, the LHRH antagonist is a nona- or decapeptide analog of LHRH that binds to LHRH receptors but does not activate gonadotropin release. LHRH antagonists include (but are not limited to) LHRH analogs having the structure of Formula I:

a-b-c-d-e-f-g-h-Pro-j (I)
1 2 3 4 5 6 7 8 9 10

or a pharmaceutically acceptable salt thereof, wherein:

a is an amino acyl residue selected from the group consisting of either the D- or the L- isomer of:
N-Ac-D,L-$\Delta^{3,4}$-prolyl,
N-Ac-D,L-prolyl,
N-Ac-D,L-alkylprolyl,
N-Ac-D,L-phenylalanyl,
N-Ac-D,L-p-chlorophenylalanyl,
N-Ac-D,L-seryl,
N-Ac-D,L-threonyl,
N-Ac-D,L-alanyl,
N-Ac-3-(1-naphthyl)-D,L-alanyl,
N-Ac-3-(2-naphthyl)-D,L-alanyl,
N-Ac-D,L-p-fluorophenylalanyl,
N-Ac-3-(2,4,6-trimethylphenyl)-D,L-alanyl, and N-Ac-3-(4-trifluoromethylphenyl)-D,L-alanyl;
b is an amino acyl residue selected from the group consisting of:
D-phenylalanyl,
D-p-chlorophenylalanyl,
D-p-bromophenylalanyl,

D-p-fluorophenylalanyl,
D-p-nitrophenylalanyl,
2,2-diphenylglycyl,
3-(3,4,5-trimethoxyphenyl)-D-alanyl,
3-(2,4,6-trimethylphenyl)-D-alanyl,
D-α-methyl-p-chlorophenylalanyl, and
3-(2-naphthyl)-D-alanyl;
c is an amino acyl residue selected from the group consisting of:
D-tryptophanyl,
D-phenylalanyl,
D-pentamethylphenylalanyl,
3-(3-pyridyl)-D-alanyl,
3-(1-naphthyl)-D-alanyl, and
3-(2-naphthyl)-D-alanyl;
d is an amino acyl residue selected from the group consisting of:
L-seryl, and
D-alanyl;
e is an amino acyl residue selected from the group consisting of:
L-phenylalanyl,
L-tyrosyl,
3-(3-pyridyl)-alanyl,
arginyl,
or h;
f is an amino acyl residue selected from the group consisting of:
3-(2-naphthyl)-D-alanyl,
3-(3-pyridyl)-D-alanyl,
D-tyrosyl,
D-tryptophanyl,
D-nicotinyl-lysyl,
pyridylacetyl-lysyl,
D-Glu(AA),
or h;
g is an amino acyl residue selected from the group consisting of:
L-leucyl,
L-norleucyl,
L-phenylalanyl,
L-tryptophanyl,
L-norvalyl and
3-(2-naphthyl)-L-alanyl;
h is an amino acyl residue selected from the group consisting of the radicals represented by the following structural formulas:

(a)

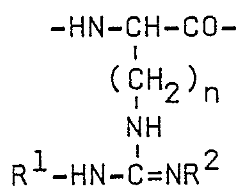

(II)

wherein
n is 1 to 5;
$R^1$ is alkyl of 1 to 6 carbon atoms or fluoroalkyl;
$R^2$ is hydrogen or $R^1$; or $R^1$-HN-C=N$R^2$ is a ring represented by the following structural formulas:

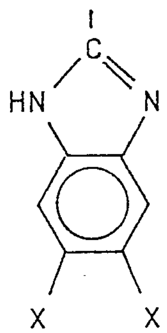
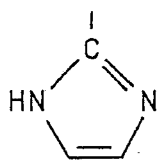
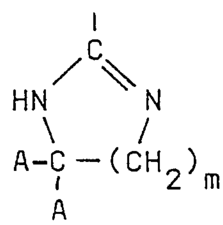

wherein m is 1 to 4; A is hydrogen or alkyl of 1 to 6 carbon atoms; and X is halo or A; and

(b)

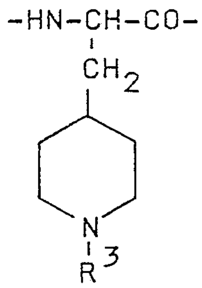
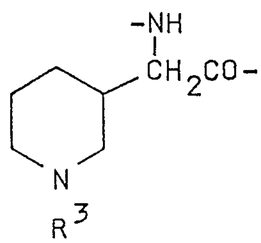

wherein $R^3$ is hydrogen, alkyl of 1 to 6 carbon atoms, phenyl or phenylloweralkyl; and
j is D-alaninamide; D-leucinamide; glycinamide; or $-NHR^4$ wherein $R^4$ is lower alkyl or $NHCONH_2$.

For convenience in describing this invention, the conventional abbreviations for the various common amino acids are used as generally accepted in the peptide art as recommended by the IUPAC-IUB Commission on Biochemical Nomenclature, Biochemistry, 11, 1726 (1972). All peptide sequences mentioned herein are written according to the generally accepted convention whereby the N-terminal amino acid is on the left and the C-terminal amino acid is on the right.

The abbreviations herein represent L-amino acids, with the exception of the achiral amino acid glycine, and with the further exception of any unnatural or natural amino acids which are achiral, or are otherwise designated as D- or D,L-.

It should be noted that when j = $NH-C(O)-NH_2$, the C-terminus is an aza-glycinamide residue.

The abbreviation D-Glu(AA) represents the anisole adduct to a D-Glu residue to form a para-methoxy-phenyl-ketone (at the carboxyl terminus of the glutamic acid side chain), i.e., Glu(pMeO-Ph).

Certain other abbreviations will be useful in describing the invention. The present invention employs replacements f amino acids in the natural LHRH peptide by amino acids which do not occur in nature. Particularly commonly employed among these are the following:

| Amino acid residue | Abbreviation |
|---|---|
| 3-(2-naphthyl)-alanyl | Nal(2) |
| 3-(p-fluorophenyl)-alanyl | pF-Phe |
| 3-(p-chlorophenyl)-alanyl | pCl-Phe |
| 3-(3-pyridyl)-alanyl | Pal(3) |
| N,N'-guanidino-dimethyl-homoarginyl | Dmh, or $hArg(Me)_2$ |
| N,N'-guanidino-(diethyl)-homoarginyl | Deh, or $hArg(Et)_2$ |
| N,N'-guanidino-(dipropyl)-homoarginyl | Dph, or $hArg(Pr)_2$ |
| N,N'-guanidino-(diisopropyl)-homoarginyl | Dih, or Arg $(iPr)_2$ |
| N,N'-guanidino-(dihexyl)-homoarginyl | Dhh, or $hArg(hexyl)_2$ |
| N,N'-guanidino-(ethano)-homoarginyl | Eha or $hArg(CH_2)_2$ |
| N,N'-guanidino-(propano)-homoarginyl | Pha, or $hArg(CH_2)_3$ |
| N,N'-guanidino-bis-(2,2,2-trifluoroethyl)-homoarginyl | Bth, or $hArg(CH_2CF_3)_2$ |
| N-guanidino-(ethyl)-homoarginyl | Meh, or hArg(Et) |
| N-guanidino-(propyl)-homoarginyl | Prh, or hArg(propyl) |
| N-guanidino-(isopropyl)-homoarginyl | Iph, or hArg(iPr) |

| Amino acid residue | Abbreviation |
|---|---|
| N-guanidino-(butyl)-homoarginyl | Mbh, or hArg(Bu) |
| N,N'-guanidino-(dicyclohexyl)-homoarginyl | Dch, or hArg(cyclohexyl)$_2$ |
| N-guanidino-(heptyl)-homoarginyl | Hha, or hArg(heptyl) |
| N-guanidino-(ethyl)-arginyl | Mea, or Arg(Et) |
| N,N'-guanidino-(diisopropyl)-arginyl | Dia, or Arg(iPr)$_2$ |
| N,N'-guanidino-(dicyclohexyl)-arginyl | Dca, or Arg(cyclohexyl)$_2$ |
| 3-(3-piperidyl)-alanyl | 3-Pia |
| 3-(4-piperidyl)-alanyl | 4-Pia |
| 3-(($N^{\varepsilon}$-methyl)piperid-4-yl)-alanyl | Mpa |
| 3-(($N^{\varepsilon}$-pentyl)piperid-4-yl)-alanyl | Ppa |
| 3-(($N^{\varepsilon}$-benzyl)piperid-4-yl)-alanyl | Bpa |
| $N^{\varepsilon}$-Nicotinyl-D-lysyl | Lys(Nic) |
| $N^{\varepsilon}$-(3-Pyridyl)acetyl-D-lysyl | Lys(pyridylacetyl) |
| 3-(2,4,6-trimethylphenyl)alanyl | Tmp |
| 2,2-diphenylglycyl | Dpg |

As used herein, the term "pharmaceutically acceptable salts" refers to salts that retain the desired biological activity of the parent compound and do not impart any undesired toxicological effects. Examples of such salts are (a) acid addition salts formed with inorganic acids, for example hydrochloric acid, hydrobromic acid, sulfuric acid, phosphoric acid, nitric acid and the like; and salts formed with organic acids such as, for example, acetic acid, oxalic acid, tartaric acid, succinic acid, maleic acid, fumaric acid, gluconic acid, citric acid, malic acid, ascorbic acid, benzoic acid, tannic acid, pamoic acid, alginic acid, polyglutamic acid, naphthalenesulfonic acids, naphthalenedisulfonic acids, polygalacturonic acid; (b) base addition salts formed with polyvalent metal cations such as zinc, calcium, bismuth, barium, magnesium, aluminum, copper, cobalt, nickel, cadmium, and the like; or with an organic cation formed from N,N′-dibenzylethylene-diamine or ethylenediamine; or (c) combinations, of (a) and (b), e.g., a zinc tannate salt and the like.

The term "lower alkyl" refers to a straight or branched chain saturated hydrocarbon radical having from 1 to 4 carbon atoms such as, for example, methyl, ethyl, n-propyl, isopropyl, n-butyl, isobutyl, sec-butyl and tert-butyl. "Alkyl of 1 to 6 carbon atoms" encompasses the same substituents as lower alkyl but in addition includes radicals having 5 or 6 carbon atoms such as, for example, n-pentyl, n-hexyl or branched 5 or 6 carbon membered radicals. "Alkyl of 1 to 12 carbon atoms" comprises a hydrocarbon radical of 1 to 12 carbon atoms including the radicals noted above, except that the radicals may have up to 12 carbon atoms.

Fluoroalkyl refers to lower alkyl substituted with 1 to 5 fluorine atoms, for example $CF_3CH_2$-, $CF_3$-, $CF_3CF_2CH_2$-, and the like.

Halo refers to fluoro, chloro or bromo.

The abbreviation "N-Ac" refers specifically to the N-acetyl protecting group, i.e., an acetyl group attached to a terminal amino acid residue on the amine nitrogen, in conformance with generally accepted nomenclature.

19-nor progestational agents are defined as steroids lacking a methyl group at the 19-position of the steroid skeleton, and possessing progestational activity that can be ascertained in assay procedures such as the Clauberg test, McPhail test, pregnancy support, delay of parturition, decidual cell reaction, etc. Particular examples include norethindrone and its acetate, ethynodiol diacetate, norethynodrel, norgestrel and lynestrenol.

As used herein, the term "a pharmaceutical composition in powder form" means a pharmaceutical composition that is in the form of a powder. The powder form consists of particles, preferably of which at least 75% are less than 100 microns in diameter, more preferably between 60 microns and 100 microns.

The term "nasal administration" means a systemic form of administration of an LHRH antagonist with or without a 19-norprogestational agent, whereby a therapeutically effective amount of the active ingredient(s), for example in powdered form, is propelled or otherwise introduced into the nasal passages of a human female such that it contacts the nasal mucosa.

The term "water-soluble polysaccharide" refers to any high-molecular weight polysaccharide (i.e. with a molecular weight of over 2000) that is soluble in water and is pharmaceutically acceptable for nasal application. Examples include microcrystalline cellulose, cellulose derivatives, dextran, and the like. A particularly preferred water-soluble polysaccharide is dextran, especially dextran T70.

The term "dextran" refers to polysaccharides that are composed exclusively of α-D-glucopyranosyl units, differing only in the degree of branching the chain length. For example, "dextran T40" refers to a mixture of such polysaccharides with an average molecular weight of 40,000, and "dextran T70" refers to a mixture of such polysaccharides with an average molecular weight of 70,000. Dextran T70 may be purchased from Pharmacia Fine Chemicals (2202 N. Bartlett Ave., Milwaukee, WI, 53202, U.S.A.).

The term "intimate admixture" is intended to indicate that the LHRH antagonist (with or without the progestational agent) and the high molecular weight, water soluble polysaccharide are intimately admixed such that the components are substantially uniformly distributed within each other. Notwithstanding the use of the term "intimate", the mixture will not necessarily be entirely homogeneous throughout, but may show some variation in the relative concentrations of the two essential components of the mixture.

The term "therapeutically effective" as used herein refers to a rate and means of drug administration of a polypeptide or 19-nor progestational agent which provides polypeptide plasma levels which are effective to achieve the desired pharmacological result. For example, if the plasma level of polypeptide required to achieve and maintain testosterone suppression in human males with a j particular LHRH analog is approximately 10 ng/ml, a therapeutically effective dose would be one which provides average plasma levels of that drug at or above 10 ng/ml.

## Preferred Embodiments

While the invention in its broadest terms relates to the combination of an effective amount of an LHRH antagonist with a menopausal-symptom-alleviating amount of a 19-nor progestational agent, certain antagonists and progestational agents are preferred.

### Preferred Antagonists

With respect to the antagonists, it should be understood that the replacement of the L-histidyl residue at position 2 in LHRH with one of the residues herein specified is a necessary requirement to obtain antagonist activity. However, the replacement of the glycyl residue at position 6 in LHRH with one of the residues specified herein gives a dramatic enhancement of the antagonist effect. The substitutions disclosed herein at positions 1, 2, 3, 5, 7 and 10 are further helpful in enhancing the antagonist activity. Moreover, the substitution specified herein at position 8 provides a profound diminution in the histamine releasing potency of the analogs when the amino acid at positions 6 is other than Arg.

Accordingly, the preferred antagonists used in the composition and methods of the invention are those which include a substitution at position 8, when the amino acid at position 6 is other than Arg. Specifically, the preferred antagonists are compounds of the formula

$$\underline{a}-\underline{b}-\underline{c}-\text{Ser}-\underline{e}-\underline{f}-\underline{g}-\underline{h}-\text{Pro}-\underline{j} \qquad \text{(IA)}$$

1 2 3 4 5 6 7 8 9 10

or a pharmaceutically acceptable salt thereof, wherein:

a is an amino acyl residue selected from the group consisting of either the D- or the L- isomer of: N-Ac-D,L-$\Delta^{3,4}$-prolyl, N-Ac-D,L-prolyl, N-Ac-D,L-phenylalanyl, N-Ac-D,L-p-chlorophenylalanyl, N-Ac-D,L,-p-fluorophenylalanyl, N-Ac-3-(1-naphthyl)-D,L-alanyl, N-Ac-3-(2-naphthyl)-D,L-alanyl, and N-Ac-3-(2,4,6-trimethylphenyl)-D,L-alanyl;

b is an amino acyl residue selected from the group consisting of D-phenylalanyl, D-p-chlorophenylalanyl, D-p-fluorophenylalanyl, D-p-nitrophenylalanyl, 2,2-diphenylglycyl, D-α-methyl-p-chlorophenylalanyl and 3-(2-naphthyl)-D-alanyl;
c is an amino acyl residue selected from the group consisting of D-tryptophanyl, D-phenylalanyl, 3-(3-pyridyl)-D-alanyl, and 3-(2-naphthyl)-D-alanyl;
e is an amino acyl residue selected from the group consisting of L-phenylalanyl, L-tyrosyl, and 3-(3-pyridyl)-alanyl, arginyl, or h;
f is 3-(2-naphthyl)-D-alanyl, 3-(3-pyridyl)-D-alanyl, D-tyrosyl, D-tryptophanyl, D-nicotinyl-lysyl, pyridylacetyl-lysyl, D-Glu(AA) or h;
g is an amino acyl residue selected from the group consisting of L-leucyl, L-norleucyl, L-phenylalanyl, L-tryptophanyl, and 3-(2-naphthyl)-L-alanyl;
h is an amino acyl residue selected from the group consisting of the radicals represented by the following structural formulas:

(a)

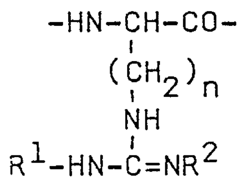 (II)

wherein
n is 1 to 5;
$R^1$ is alkyl of 1 to 6 carbon atoms or fluoroalkyl;
$R^2$ is hydrogen or $R^1$; or $R^1$-HN-C=$NR^2$ is a ring represented by the following structural formulas:

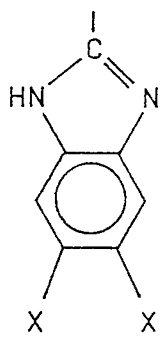

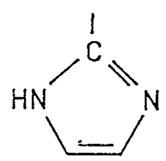

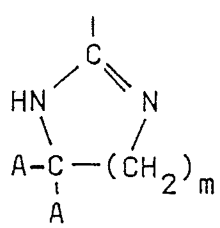

wherein m is 1 to 4; A is hydrogen or alkyl of 1 to 6 carbon atoms; and X is halo or A; and

(b)

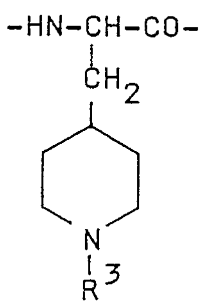

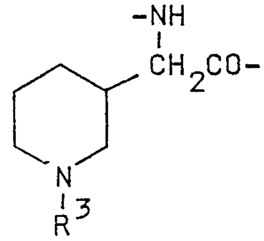

wherein $R^3$ is hydrogen, alkyl of 1 to 6 carbon atoms, phenyl or phenylloweralkyl; and
j is D-alaninamide; D-leucinamide; glycinamide; or $-NHR^4$ wherein $R^4$ is lower alkyl or $NHCONH_2$.

Antagonists which form part of the more preferred embodiments of the present invention are those of

Formula (I) wherein a is N-Ac-D-Nal(2) or N-Ac-D-pCl-Phe; b is D-pF-Phe or D-pCl-Phe; c is D-Trp, D-Nal(2) or Pal(3); d is Ser; e is Pal(3), Tyr, Arg, Deh, Mbh, Bth, or Pha; f is D-Trp, D-Tyr, D-Nal(2), D-Pal(3), D-Deh, D-Mbh, D-Pha or D-Bth; g is Leu or Phe; h is Deh, Bth, Mbh, or Pha; and j is D-$AlaNH_2$ or $GlyNH_2$.

Most preferred substitution patterns are those of Formula (I) wherein:

a is N-Ac-D-Nal(2);
b is D-pCl-Phe;
c is D-Trp or D-Pal(3);
d is Ser;
e is Tyr, Arg, Deh, Mbh, Bth or Pha;
f is D-Trp, D-Pal(3), D-Nal(2), D-Tyr, D-Deh, D-Mbh, D-Bth or D-Pha;
g is Leu;
h is Deh, Mbh, Bth or Pha; and
j is D-$AlaNH_2$.

There are three particularly preferred subclasses within this mot preferred class:

1. When e is Tyr, f can be any one of either (a) the hydrophobic residues D-Trp, D-Pal(3), D-Nal(2) or D-Tyr, but most preferably D-Trp or D-Pal(3), or (b) the residues D-Deh, D-Mbh, D-Bth or D-Pha;
2. When e is Arg, f is preferably one of the hydrophobic residues listed in 1(a) above, and most preferably D-Tyr;
3. When e is any of Deh, Mbh, Bth or Pha, f is most preferably D-Tyr or D-Pal(3), but D-Nal(2) and D-Trp are also preferred. In each of the foregoing subclasses, h is Deh, Mbh, Bth or Pha. Particularly preferred are Bth and Deh, and most particularly, Deh.

Thus, examples of the more preferred substitution patterns are:

N-Ac-D-Nal(2)-D-pCl-Phe-c-Ser-Tyr-c-Leu-h-Pro-D-$AlaNH_2$, wherein c is D-Trp or D-Pal(3) and h is Deh, Mbh, Bth or Pha;

N-Ac-D-Nal(2)-D-pCl-Phe-c-Ser-Tyr-f-Leu-h-Pro-D-$AlaNH_2$, wherein c is D-Trp or D-Pal(3), f is D-Deh, D-Bth, D-Mbh or D-Pha, and h is the L-form of f as defined in this paragraph;

N-Ac-D-Nal(2)-D-pCl-Phe-c-Ser-Arg-f-Leu-h-Pro-D-$AlaNH_2$, wherein c is D-Trp or D-Pal(3), f is D-Trp, D-Pal(3), D-Nal(2), or D-Tyr, and h is Deh, Mbh, Bth or Pha; and

N-Ac-D-Nal(2)-D-pCl-Phe-c-Ser-e-f-Leu-G-Pro-D-$AlaNH_2$, wherein c is D-Trp or D-Pal(3), e and h are independently Deh, Bth, Mbh or Pha, and f is D-Tyr, D-Nal(2), D-Trp or D-Pal(3).

Additional preferred embodiments include antagonists of the following formulas:

N-Ac-D-Nal(2)-D-pCl-Phe-D-Trp-Ser-Tyr-f-Leu-h-Pro-D-$AlaNH_2$, wherein f is D-Trp, D-Tyr, or D-Nal(2) and h is Deh, Bth, Mbh, or Pha;

N-Ac-D-Nal(2)-D-pCl-Phe-c-Ser-e-f-Leu-h-Pro-D-$AlaNH_2$, wherein e and h are independently Deh, Bth, Mbh or Pha, and both c and f are independently D-Trp or D-Pal(3); and

N-Ac-D-Nal(2)-D-pCl-Phe-D-Pal(3)-Ser-Tyr-D-Pal(3)-Leu-h-Pro-D-$AlaNH_2$, wherein h is Deh, Bth, Mbh, or Pha.

Exemplary preferred antagonists are:

N-Ac-D-Nal(2)-D-pCl-Phe-D-Pal(3)-Ser-Tyr-D-Pal(3)-Leu-Deh-Pro-D-$AlaNH_2$;
N-Ac-D-Nal(2)-D-pCl-Phe-D-Pal(3)-Ser-Tyr-D-Pal(3)-Leu-Bth-Pro-D-$AlaNH_2$;
N-Ac-D-Nal(2)-D-pCl-Phe-D-Pal(3)-Ser-Tyr-D-Pal(3)-Leu-Mbh-Pro-D-$AlaNH_2$;
N-Ac-D-Nal(2)-D-pCl-Phe-D-Pal(3)-Ser-Tyr-D-Pal(3)-Leu-Pha-Pro-D-$AlaNH_2$;
N-Ac-D-Nal(2)-D-pCl-Phe-D-Trp-Ser-Tyr-D-Trp-Leu-Deh-Pro-D-$AlaNH_2$;
N-Ac-D-Nal(2)-D-pCl-Phe-D-Trp-Ser-Tyr-D-Trp-Leu-Bth-Pro-D-$AlaNH_2$;
N-Ac-D-Nal(2)-D-pCl-Phe-D-Trp-Ser-Tyr-D-Trp-Leu-Mbh-Pro-D-$AlaNH_2$;
N-Ac-D-Nal(2)-D-pCl-Phe-D-Trp-Ser-Tyr-D-Trp-Leu-Pha-Pro-D-$AlaNH_2$;
N-Ac-D-Nal(2)-D-pCl-Phe-D-Trp-Ser-Tyr-D-Deh-Leu-Deh-Pro-D-$AlaNH_2$;
N-Ac-D-Nal(2)-D-pCl-Phe-D-Trp-Ser-Tyr-D-Mbh-Leu-Mbh-Pro-D-$AlaNH_2$;
N-Ac-D-Nal(2)-D-pCl-Phe-D-Trp-Ser-Tyr-D-Bth-Leu-Bth-Pro-D-$AlaNH_2$;
N-Ac-D-Nal(2)-D-pCl-Phe-D-Trp-Ser-Tyr-D-Pha-Leu-Pha-Pro-D-$AlaNH_2$;
N-Ac-D-Nal(2)-D-pCl-Phe-D-Pal(3)-Ser-Tyr-D-Deh-Leu-Deh-Pro-D-$AlaNH_2$;
N-Ac-D-Nal(2)-D-pCl-Phe-D-Pal(3)-Ser-Tyr-D-Mbh-Leu-Mbh-Pro-D-$AlaNH_2$;
N-Ac-D-Nal(2)-D-pCl-Phe-D-Pal(3)-Ser-Tyr-D-Bth-Leu-Bth-Pro-D-$AlaNH_2$;
N-Ac-D-Nal(2)-D-pCl-Phe-D-Pal(3)-Ser-Tyr-D-Pha-Leu-Pha-Pro-D-$AlaNH_2$;
N-Ac-D-Nal(2)-D-pCl-Phe-D-Trp-Ser-Arg-D-Trp-Leu-Deh-Pro-D-$AlaNH_2$;
N-Ac-D-Nal(2)-D-pCl-Phe-D-Trp-Ser-Arg-D-Trp-Leu-Bth-Pro-D-$AlaNH_2$;
N-Ac-D-Nal(2)-D-pCl-Phe-D-Trp-Ser-Arg-D-Trp-Leu-Mbh-Pro-D-$AlaNH_2$;
N-Ac-D-Nal(2)-D-pCl-Phe-D-Trp-Ser-Arg-D-Trp-Leu-Pha-Pro-D-$AlaNH_2$;
N-Ac-D-Nal(2)-D-pCl-Phe-D-Pal(3)-Ser-Arg-D-Pal(3)-Leu-Bth-Pro-D-$AlaNH_2$;
N-Ac-D-Nal(2)-D-pCl-Phe-D-Pal(3)-Ser-Arg-D-Pal(3)-Leu-Mbh-Pro-D-$AlaNH_2$;
N-Ac-D-Nal(2)-D-pCl-Phe-D-Pal(3)-Ser-Arg-D-Pal(3)-Leu-Deh-Pro-D-$AlaNH_2$;
N-Ac-D-Nal(2)-D-pCl-Phe-D-Pal(3)-Ser-Arg-D-Pal(3)-Leu-Pha-Pro-D-$AlaNH_2$;
N-Ac-D-Nal(2)-D-pCl-Phe-D-Trp-Ser-Arg-D-Tyr-Leu-Deh-Pro-D-$AlaNH_2$;
N-Ac-D-Nal(2)-D-pCl-Phe-D-Trp-Ser-Arg-D-Tyr-Leu-Bth-Pro-D-$AlaNH_2$;
N-Ac-D-Nal(2)-D-pCl-Phe-D-Trp-Ser-Arg-D-Tyr-Leu-Mbh-Pro-D-$AlaNH_2$;

N-Ac-D-Nal(2)-D-pCl-Phe-D-Trp-Ser-Arg-D-Tyr-Leu-Pha-Pro-D-Ala$NH_2$;
N-Ac-D-Nal(2)-D-pCl-Phe-D-Pal(3)-Ser-Arg-D-Tyr-Leu-Bth-Pro-D-Ala$NH_2$;
N-Ac-D-Nal(2)-D-pCl-Phe-D-Pal(3)-Ser-Arg-D-Tyr-Leu-Mbh-Pro-D-Ala$NH_2$;
N-Ac-D-Nal(2)-D-pCl-Phe-D-Pal(3)-Ser-Arg-D-Tyr-Leu-Deh-Pro-D-Ala$NH_2$;
N-Ac-D-Nal(2)-D-pCl-Phe-D-Pal(3)-Ser-Arg-D-Tyr-Leu-Pha-Pro-D-Ala$NH_2$;
N-Ac-D-Nal(2)-D-pCl-Phe-D-Trp-Ser-Deh-D-Tyr-Leu-Deh-Pro-D-Ala$NH_2$;
N-Ac-D-Nal(2)-D-pCl-Phe-D-Trp-Ser-Mbh-D-Tyr-Leu-Mbh-Pro-D-Ala$NH_2$;
N-Ac-D-Nal(2)-D-pCl-Phe-D-Trp-Ser-Bth-D-Tyr-Leu-Bth-Pro-D-Ala$NH_2$;
N-Ac-D-Nal(2)-D-pCl-Phe-D-Trp-Ser-Pha-D-Tyr-Leu-Pha-Pro-D-Ala$NH_2$;
N-Ac-D-Nal(2)-D-pCl-Phe-D-Trp-Ser-Deh-D-Trp-Leu-Deh-Pro-D-Ala$NH_2$;
N-Ac-D-Nal(2)-D-pCl-Phe-D-Trp-Ser-Mbh-D-Trp-Leu-Mbh-Pro-D-Ala$NH_2$;
N-Ac-D-Nal(2)-D-pCl-Phe-D-Trp-Ser-Bth-D-Trp-Leu-Bth-Pro-D-Ala$NH_2$;
N-Ac-D-Nal(2)-D-pCl-Phe-D-Trp-Ser-Pha-D-Trp-Leu-Pha-Pro-D-Ala$NH_2$;
N-Ac-D-Nal(2)-D-pCl-Phe-D-Pal(3)-Ser-Deh-D-Pal(3)-Leu-Deh-Pro-D-Ala$NH_2$;
N-Ac-D-Nal(2)-D-pCl-Phe-D-Pal(3)-Ser-Mbh-D-Pal(3)-Leu-Mbh-Pro-D-Ala$NH_2$;
N-Ac-D-Nal(2)-D-pCl-Phe-D-Pal(3)-Ser-Bth-D-Pal(3)-Leu-Bth-Pro-D-Ala$NH_2$;
N-Ac-D-Nal(2)-D-pCl-Phe-D-Pal(3)-Ser-Pha-D-Pal(3)-Leu-Pha-Pro-D-Ala$NH_2$;
N-Ac-D-Nal(2)-D-pCl-Phe-D-Trp-Ser-Deh-D-Nal(2)-Leu-Deh-Pro-D-Ala$NH_2$;
N-Ac-D-Nal(2)-D-pCl-Phe-D-Trp-Ser-Mbh-D-Nal(2)-Leu-Mbh-Pro-D-Ala$NH_2$;
N-Ac-D-Nal(2)-D-pCl-Phe-D-Trp-Ser-Bth-D-Nal(2)-Leu-Bth-Pro-D-Ala$NH_2$;
N-Ac-D-Nal(2)-D-pCl-Phe-D-Trp-Ser-Pha-D-Nal(2)-Leu-Pha-Pro-D-Ala$NH_2$;
N-Ac-D-Nal(2)-D-pCl-Phe-D-Pal(3)-Ser-Pal(3)-D-Pal(3)-Leu-Deh-Pro-D-Ala$NH_2$;
N-Ac-D-Nal(2)-D-pCl-Phe-D-Pal(3)-Ser-Pal(3)-D-Pal(3)-Leu-Bth-Pro-D-Ala$NH_2$;
N-Ac-D-Nal(2)-D-pCl-Phe-D-Pal(3)-Ser-Pal(3)-D-Pal(3)-Leu-Pha-Pro-D-Ala$NH_2$;
N-Ac-D-Nal(2)-D-pCl-Phe-D-Pal(3)-Ser-Pal(3)-D-Pal(3)-Leu-Mbh-Pro-D-Ala$NH_2$;
N-Ac-D-Nal(2)-D-pCl-Phe-D-Pal(3)-Ser-Tyr-D-Mbh-Leu-Bth-Pro-D-Ala$NH_2$;
N-Ac-D-Nal(2)-D-pCl-Phe-D-Trp-Ser-Tyr-D-Mbh-Leu-Bth-Pro-D-Ala$NH_2$;
N-Ac-D-Nal(2)-D-pF-Phe-D-Pal(3)-Ser-Tyr-D-Pal(3)-Leu-Deh-Pro-D-Ala$NH_2$;
N-Ac-D-Nal(2)-D-pF-Phe-D-Pal(3)-Ser-Tyr-D-Pal(3)-Leu-Bth-Pro-D-Ala$NH_2$;
N-Ac-D-Nal(2)-D-pF-Phe-D-Trp-Ser-Tyr-D-Trp-Leu-Deh-Pro-D-Ala$NH_2$;
N-Ac-D-Nal(2)-D-pF-Phe-D-Trp-Ser-Tyr-D-Trp-Leu-Bth-Pro-D-Ala$NH_2$;
N-Ac-D-Nal(2)-D-pF-Phe-D-Trp-Ser-Tyr-D-Deb-Leu-Deh-Pro-D-Ala$NH_2$;
N-Ac-D-Nal(2)-D-pF-Phe-D-Trp-Ser-Tyr-D-Bth-Leu-Bth-Pro-D-Ala$NH_2$;
N-Ac-D-Nal(2)-D-pf-Phe-D-Pal(3)-Ser-Tyr-D-Deh-Leu-Deh-Pro-D-Ala$NH_2$;
N-Ac-D-Nal(2)-D-pF-Phe-D-Pal(3)-Ser-Tyr-D-Bth-Leu-Bth-Pro-D-Ala$NH_2$;
N-Ac-D-Nal(2)-D-pF-Phe-D-Trp-Ser-Arg-D-Trp-Leu-Deh-Pro-D-Ala$NH_2$;
N-Ac-D-Nal(2)-D-pF-Phe-D-Trp-Ser-Arg-D-Trp-Leu-Bth-Pro-D-Ala$NH_2$;
N-Ac-D-Nal(2)-D-pF-Phe-D-Pal(3)-Ser-Arg-D-Pal(3)-Leu-Bth-Pro-D-Ala$NH_2$;
N-Ac-D-Nal(2)-D-pF-Phe-D-Pal(3)-Ser-Arg-D-Pal(3)-Leu-Deh-Pro-D-Ala$NH_2$;
N-Ac-D-Nal(2)-D-pF-Phe-D-Trp-Ser-Deh-D-Tyr-Leu-Deh-Pro-D-Ala$NH_2$; and
N-Ac-D-Nal(2)-D-pF-Phe-D-Trp-Ser-Bth-D-Tyr-Leu-Bth-Pro-D-Ala$NH_2$;

The scope of the instant invention also includes peptides that may not necessarily fall within the aforementioned preferred classes, such as:

N-Ac-D-Nal(2)-D-pCl-Phe-D-Trp-Ser-Tyr-D-Tyr-Leu-Deh-Pro-D-Ala$NH_2$;
N-Ac-D-Nal(2)-D-pCl-Phe-D-Trp-Ser-Tyr-D-Tyr-Leu-Mbh-Pro-D-Ala$NH_2$;
N-Ac-D-Nal(2)-D-pCl-Phe-D-Trp-Ser-Tyr-D-Tyr-Leu-Bth-Pro-D-Ala$NH_2$;
N-Ac-D-Nal(2)-D-pCl-Phe-D-Trp-Ser-Tyr-D-Tyr-Leu-Pha-Pro-D-Ala$NH_2$;
N-Ac-D-Nal(2)-D-pCl-Phe-D-Pal(3)-Ser-Tyr-D-Nal(2)-Leu-Deh-Pro-D-Ala$NH_2$;
N-Ac-D-Nal(2)-D-pCl-Phe-D-Pal(3)-Ser-Tyr-D-Nal(2)-Leu-Mbh-Pro-D-Ala$NH_2$;
N-Ac-D-Nal(2)-D-pCl-Phe-D-Pal(3)-Ser-Tyr-D-Nal(2)-Leu-Bth-Pro-D-Ala$NH_2$;
N-Ac-D-Nal(2)-D-pCl-Phe-D-Pal(3)-Ser-Tyr-D-Nal(2)-Leu-Pha-Pro-D-Ala$NH_2$
N-Ac-D-Nal(2)-D-αMe,pCl-Phe-D-Pal(3)-Ser-Arg-D-Pal(3)-Leu-Bth-Pro-D-Ala$NH_2$;
N-Ac-D-Nal(2)-D-αMe,pCl-Phe-D-Pal(3)-Ser-Tyr-D-Pal(3)-Leu-Bth-Pro-D-Ala$NH_2$;
N-Ac-D-Nal(2)-D-αMe,pCl-Phe-D-Trp-Ser-Arg-D-Trp-Leu-Bth-Pro-D-Ala$NH_2$;
N-Ac-D-Nal(2)-D-pCl-Phe-D-Pal(3)-Ser-Tyr-D-Glu(AA)-Leu-Bth-Pro-D-Ala$NH_2$;
N-Ac-D-Nal(2)-D-pCl-Phe-D-Pal(3)-Ser-Arg-D-Glu(AA)-Leu-Bth-Pro-D-Ala$NH_2$;
N-Ac-D-Nal(2)-D-pCl-Phe-D-Trp-Ser-Arg-D-Glu(AA)-Leu-Bth-Pro-D-Ala$NH_2$;
N-Ac-D-pCl-Phe-D-Phe-D-Phe-Ser-Phe-D-Lys(Nic)-Nle-Bth-Pro-Gly$NH_2$;
N-Ac-$\Delta^{3,4}$Pro-D-Nal(2)-D-Pal(3)-Ser-Pal(3)-D-Lys(pyridylacetyl)-Phe-Mpa-D-Ala$NH_2$;
N-Ac-Pro-D-p$NO_2$-Phe-D-Trp-Ser-Phe-D-Lys(Nic)-Leu-Ppa-Pro-D-Leu$NH_2$;
N-Ac-D-pF-Phe-D-pF-Phe-D-Trp-Ser-Tyr-D-Tyr-Trp-Bth-Pro-AzaGly$NH_2$;
N-Ac-D-Nal(1)-Dpg-D-Pal(3)-Ser-Tyr-D-Pal(3)-Nal(2)-Bth-Pro-D-Ala$NH_2$;and
N-Ac-D-Tmp-D-pF-Phe-D-Pal(3)-Ser-Tyr-D-Lys(Nic)-Nal(2)-Bth-Pro-NHEt.

It is generally preferred that the a, b, c, f and j amino acid residues be in the form of the D-isomer; and that the e, g and h residues be in the form of the L-isomer. This stereochemistry is to be understood to be

represented where not otherwise specified.

At the present time, the two most preferred LHRH antagonists are:
N-Ac-D-Nal(2)-D-pCl-Phe-D-Pal(3)-Ser-Tyr-D-Pal(3)-Leu-Deh-Pro-D-AlaN$H_2$; and
N-Ac-D-Nal(2)-D-pCl-Phe-D-Pal(3)-Ser-Tyr-D-Deh-Leu-Deh-Pro-D-AlaN$H_2$.

In all of the above embodiments, the antagonist may also be prepared as a corresponding pharmaceutically acceptable salt.

## Preferred Progestational Agents

While any 19-nor progestational agent can be used in the broadest scope of this invention, certain progestational agents are preferred. The preferred agents are norethindrone, norethindrone acetate, ethynodiol diacetate, norethynodrel, norgestrel, and lynestrenol. Of these, the more preferred are norethindrone and norethindrone acetate. The most preferred progestational agent is norethindrone.

## Assary Procedures

The compounds of this invention and, particularly, the salts thereof, exhibit surprisingly potent and long lasting LHRH antagonist activity.

A primary measure of LHRH antagonist potency is the ability to inhibit ovulation in rats, as assayed by the procedure of Corbin, A. and Beattie, C. W., Endocrine Res. Commun., 2:1 (1975).

The ability to cause histamine release from rat peritoneal mast cells in vitro may be assessed as per Sydbom and Terenius, Agents and Actions, 16, 269 (1985) or Siraganian, et al., Manual of Clinical Immunology, 2d Ed, N. E. Rose and M. Friedman, Eds., Amer. Soc. Microbiol., Washington, D.C., 1980, p 808.

Other bioassays which may be used for LHRH antagonists and for the compounds of the present invention are:

(a) inhibition of LHRH induced FSH and LH release in the rat, in vivo; Vilchez-Martinez, J.A., et al, Endocrinology, 96: 1130 (1975); and,

(b) inhibition of LH and FSH release by dispersed anterior pituitary cell cultures as measured by radioimmunoassay. (Vale, W., et al, Endocrinology 91: 562 (1972).

(c) inhibition of gonadotropin levels in castrated rat and dog (Petrie et al., Male Contraception, Harper and Row, Philadelphia (1985), p.361).

(c) suppression of testosterone levels in male dogs (Vickery et al., LHRH and Its Analogs: Contraceptive and Therapeutic Applications Part 2, MTP Press Boston (1987) p. 517).

## Antagonist Effects and Utilities

This invention is useful for providing antagonist effects while inhibiting menopausal-type symptoms such as vasomotor symptoms (i.e., "hot flashes") and bone density loss symptoms (i.e, "osteoporosis").

The following utilities flow from the antagonist effect of the compounds herein:

-female contraception;
-ovulation prevention or delay;
-therapy for precocious puberty;
-therapy for uterine leiomyomata;
-therapy for endometriosis;
-therapy for mammary tumors and cysts
-therapy for polycystic ovary syndrome/ disease;
-therapy for uterine carcinoma;
-therapy for diseases which result from excessive gonadal hormone production in females;
-and other uses as set forth in Vickery, B. H., Endocrine Reviews, 7:115 (1986), which is fully incorporated by reference herein.

The aspect of the present invention which relates to particular uses for the above-described antagonists and progestational agents in combination is most particularly directed to: inhibition of ovulation; treatment of endometriosis, uterine leiomyomata, breast cancer, or polycystic ovarian disease in a human female; and suppression of isosexual, true (idiopathic) precocious puberty (i.e., precocious puberty of hypothalamic origin) in females.

In the practice of this invention, a therapeutically effective amount of an LHRH antagonist and a menopausal-symptom-alleviating amount of a 19-nor progestational agent are administered concurrently, either as two medications to be taken together, or as a single medication to be taken as a single pharmaceutical composition containing both ingredients and optionally a pharmaceutical excipient, to a subject in need of, or desiring, such treatment. These compounds or compositions may be administered by any of a variety of routes depending upon the specific end use, including orally, parenterally (including subcutaneous, intramuscular and intravenous administration), vaginally (particularly for contraception), rectally, buccally (including sublingually), transdermally or intranasally. For best results, the antagonist is not administered orally; intranasal administration of the antagonist is preferred. Accordingly, if the antagonist and the progestagen are administered together as a single pharmaceutical composition, nasal administration

would be the method of choice as a general rule. Alternatively, the two active ingredients could be formulated into a single injectable formulation. If the antagonist and the progestagen are administered as two discrete pharmaceutical compositions, then the progestagen is preferably orally administered; while the antagonist is preferably administered nasally, or can be administered by injection. However, the most suitable route of administration in any given case will depend upon the use, particular active ingredient, the subject involved, and the judgment of the medical practitioner. The compound or composition may also be administered by means of controlled-release, depot implant or injectable formulations as described more fully herein below.

In general, for the uses herein above described, it is expedient to administer the antagonist in amounts between about 0.001 and 5 mg/kg body weight, and the progestagen in amounts between 0.007 and 0.1 mg/kg body weight. Preferably, for human therapy, the antagonist will be administered in the range of from about 0.01 to about 1 mg/kg/day while the progestagen will be administered in the range of from about 0.02 to about 0.07 mg/kg/day. This administration may be accomplished by a single administration, by distribution over several applications or by slow release in order to achieve the most effective results. For a suitable example of a delayed/sustained release implant using hydrogel, see European Patent Publication No. 246,653 (Canadian Patent Application S.N. 537691, Australian Patent Application No. 73272, New Zealand Patent Application No. 220393).

The administration of the LHRH analog and the progestational agent can be performed simultaneously, e.g., with the two components formulated together in a pharmaceutical composition; or separately, with the LHRH analog administered in one formulation and the progestational agent administered in another. For example, while it is preferred to administer the LHRH analog by a nasal route and the progestagen by tablet (orally), both components can be administered together nasally or by injection.

The exact dose and regimen for administration of these compounds and compositions will necessarily be dependent upon the needs of the individual subject being treated, the type of treatment, the degree of affliction or need and, of course, the judgment of the medical practitioner. In general, parenteral administration requires lower dosage than other methods of administration which are more dependent upon absorption.

A further aspect of the present invention relates to pharmaceutical compositions containing, in addition to the antagonist and the progestagen, a pharmaceutically acceptable, non-toxic carrier or excipient in admixture therewith. As mentioned above, such compositions may be prepared for use for parenteral (subcutaneous, intramuscular or intravenous) administration particularly in the form of liquid solutions or suspensions; for use in vaginal or rectal administration particularly in semisolid forms such as creams and suppositories; for oral or buccal administration particularly in the form of tablets or capsules; or intranasally particularly in the form of powders, nasal drops or aerosols.

The compositions may conveniently be administered in unit dosage form and may be prepared by any of the methods well-known in the pharmaceutical art, for example as described in Remington's Pharmaceutical Sciences, Seventeenth Ed., Mack Publishing Company, Easton, PA., 1985. Formulations for parenteral administration may contain as common excipients sterile water or saline, alkylene glycols such as propylene glycol, polyalkylene glycols such as polyethylene glycol, oils of vegetable origin, hydrogenated naphthalenes and the like. For buccal administration typical excipients include sugars, calcium stearate, magnesium stearate, pregelatinated starch, and the like. Formulations for vaginal or rectal administration, e.g. suppositories, may contain as excipients, for example, polyalkyleneglycols, vaseline, cocoa butter, and the like. Formulations for nasal administration may be solid an contain as excipients, for example, lactose, dextran or α-cyclodextrin, or may be aqueous or oily solutions for administration in the form of nasal drops or metered spray.

Nasal administration of the instant nona- and decapeptides is particularly preferred, and is a particularly suitable method for the pharmaceutical compositions of the invention comprising both an antagonist and a progestagen. The absorption across the nasal mucous membrane is enhanced by bile acid surfactants, such as for example, glycocholic acid, cholic acid, taurocholic acid, cholanic acid, ethocholic acid, desoxycholic acid, chenodesoxycholic acid, dehydrochloric acid, and glycodeoxy-cholic acid.

A preferred method of nasal administration is the administration of the LHRH analog formulated in powdered form in admixture with a water soluble polysaccharide excipient in powdered form. A particularly preferred polysaccharide is dextran, more particularly dextran T70. The progestational agent can optionally be included in this formulation. In a most preferred embodiment, the LHRH analog and dextran T70 intimately admixed are in the form of a powder in which at least 75% of the particles in the powder are below 100 microns in diameter, preferably between 60 microns and 100 microns, especially where substantially all of the particles in the powder are between 60 microns and 100 microns in diameter.

The relative proportions of LHRH antagonist and polysaccharide components within this preferred formulation can be varied depending on the antagonist to be administered and the dosage level desired. The antagonist may comprise up to about 40% by weight of the formulation. The precise amount will depend on such factors as the potency of the particular active agent, its physicochemical and pharmacokinetic behaviour, its stability and the condition being treated.

A preferred composition for this formulation comprises, by weight:

(a) 60 to 99.9999 percent polysaccharide; and

(b) 0.0001 to 40 percent antagonist. This particular preferred formulation is well-suited to the controlled delivery of LHRH antagonists. The amount of LHRH antagonist incorporated with the polysaccharide will preferably be 20%, or less, depending on the particular LHRH antagonist and the other factors listed above. A

presently preferred composition comprises, by weight:
(a) 80 to 99.999 percent polysaccharide; and
(b) 0.001 to 20 percent LHRH antagonist.
A more preferred composition comprises, by weight:
(a) 80 to 99.999 percent dextran T70; and
(b) 0.001 to 20 percent LHRH antagonist.
A most preferred composition comprises, by weight:
(a) 92 to 98 percent dextran T70; and
(b) 2 to 8 percent LHRH antagonist.
These powder compositions may also include an absorption enhancer. As used herein, the term "absorption enhancer" refers to a material or compound which, when incorporated in the powder mixture, causes further enhancement of the total amount of the LHRH antagonist absorbed intranasally into the systemic circulation from the powder formulation.

The preferred agents optionally used for enhancing the absorption of polypeptides across the nasal membrane from the powder formulations are bile acid surfactants, or a pharmaceutically acceptable salt thereof. Preferably, the surfactant will be an alkali metal salt of glycocholic acid, most preferably sodium glycocholate.

The amount of surfactant should be some amount which increases the absorption of LHRH antagonists over that of other surfactants which also may enhance peptide absorption to a certain degree. It has been found that such an amount is often in the range between 0.2 and 15%, more often 0.2 to 5 percent by weight/volume of the solution. It is preferred that the surfactant be present in an amount between about 0.5 to 4 percent by weight volume, conveniently about 1 percent by weight volume, preferably about 2 percent by weight volume.

A second preferred composition for the formulation comprises, by weight:
(a) 50 to 99.8999 percent polysaccharide;
(b) 0.0001 to 40 percent biologically active polypeptide; and
(c) 0.1 to 10 percent absorption enhancer.
A more preferred composition comprises, by weight:
(a) 75 to 98.999 percent dextran T70;
(b) 0.001 to 20 percent LHRH antagonist; and
(c) 1 to 5 percent bile acid surfactant.
A most preferred composition comprises, by weight:
(a) 89 to 97 percent dextran T70;
(b) 2 to 8 percent LHRH antagonist; and
(c) 1 to 3 percent sodium glycocholate.

Other materials such as preservatives, salts to achieve the tonicity value of tissue, or other additives indicated by known nasal formulation chemistry may be added to these formulations. Particularly advantageous other such materials include surfactants, suitably non-ionic surfactants such as the polysorbates, in concentrations suitably in the range 0.1 to 5, more suitably 0.25 to 2% weight volume.

It has been found that often to obtain enhanced solubility and stability, the molar ratio of bile acid to peptide is usefully $\geqq$20:1, such as $\geqq$25:1.

It is particularly desirable to deliver the compounds of the present invention to the subject over prolonged periods of time, for example, for periods of one week to one year from a single administration. Various slow release, depot implant or injectable dosage forms may be utilized. For example, a dosage form may contain a pharmaceutically acceptable non-toxic salt of the compound which has a low degree of solubility in body fluids, for example, (a) an acid addition salt with a polybasic acid such as phosphoric acid, sulfuric acid, citric acid, tartaric acid, tannic acid, pamoic acid, alginic acid, polyglutamic acid, naphthalene mono- or di-sulfonic acids, polygalacturonic acid, and the like; (b) a salt with a polyvalent metal cation such as zinc, calcium, bismuth, barium magnesium, aluminum, copper, cobalt, nickel, cadmium and the like, or with an organic cation formed from e.g., N,N′-dibenzylethylenediamine or ethylenediamine; or (c) combinations of (a) and (b) e.g. a zinc tannate salt. Additionally, the compounds of the present invention or, preferably, a relatively insoluble salt such as those just described, may be formulated in a gel, for example, an aluminum monostearate gel with, e.g. sesame oil, suitable for injection. Particularly preferred salts are zinc salts, zinc tannate salts, pamoate salts, and the like. Another type of slow release depot formulation for injection or implantation would contain the compound or salt dispersed or encapsulated in a slowly degrading, non-toxic, non-antigenic polymer such as a polylactic acid/polyglycolic acid polymer. The compounds or, preferably, relatively insoluble salts such as those described above may also be formulated in cholesterol matrix pellets, or silicone elastomer matrix systems such as implants, which are known in the art. For example, additional slow release, depot implant or injectable formulations, e.g. liposomes, are well known in the literature; see, for example, Sustained and Controlled Release Drug Delivery Systems, J. R. Robinson ed., Marcel Dekker, Inc., New York, 1978. Particular reference with respect to LHRH type compounds may be found, for example, in U.S. Patent No. 4,010,125, and in Pitt C. G., LHRH and its Analogs: Contraceptive and Therapeutic Applications, Part 2, MTP Press, Boston, 1987, p. 557.

Yet another aspect of this invention relates to a pharmaceutical package comprising an LHRH antagonist in a dosage form together with instructions prescribing co-administration of a menopausal-symptom-alleviating amount of a 19-nor-progestational agent. Alternatively, the pharmaceutical package may comprise a

19-nor-progestational agent in a menopausal-symptom-alleviating dosage form together with instructions prescribing co-administration of a suitable dose of an LHRH antagonist. Suitable instructions are package inserts, references to literature or medical publications, or other accompanying instructions.

A further aspect of this invention relates to a pharmaceutical package comprising an LHRH antagonist in a dosage form packaged in combination with a 19-nor progestational agent in a menopausal-symptom-alleviating dosage form.

Synthesis of the 19-nor-progestational agents

The 19-nor progestational agents useful in the subject invention may be prepared by known methods.

Synthesis of the Peptides

The polypeptides of the present invention may be synthesized by an techniques that are known to those skilled in the peptide art. An excellent summary of the many techniques so available may be found in J.M. Stewart and J.D. Young, Solid Phase Peptide Synthesis, W.H. Freeman Co., San Francisco, 1969, and J. Meienhofer, 7678Y

Hormonal Proteins and Peptides, Vol. 2, p. 46., Academic Press (New York), 1973 for solid phase peptide synthesis and E. Schroder and K. Lubke, The Peptides, Vol. 1, Academic Press (New York), 1965 for classical solution synthesis.

In general, these methods comprise the sequential addition of one or more amino acids or suitably protected amino acids to a growing peptide chain. Normally, either the amino or carboxyl group of the first amino acid is protected by a suitable protecting group. The protected or derivatized amino acid can then be either attached to an inert solid support or utilized in solution by adding the next amino acid in the sequence having the complimentary (amino or carboxyl) group suitably protected, under conditions suitable for forming the amide linkage. The protecting group is then removed from this newly added amino acid residue and the next amino acid (suitably protected) is then added, and so forth. After all the desired amino acids have been linked in the proper sequence, any remaining protecting groups (and any solid support) are removed sequentially or concurrently, to afford the final polypeptide. By simple modification of this general procedure, it is possible to add more than one amino acid at a time to a growing chain, for example, by coupling (under conditions which do not racemize chiral centers) a protected tripeptide with a properly protected dipeptide to form, after deprotection, a pentapeptide.

Preferred Embodiments of Synthesis

A particularly preferred method of preparing the polypeptide compounds of the present invention involves solid phase peptide synthesis.

In this particularly preferred method the α-amino function of the amino acids is protected by an acid or base sensitive group. Such protecting groups should have the properties of being stable to the conditions of peptide linkage formation, while being readily removable without destruction of the growing peptide chain or racemization of any of the chiral centers contained therein. Suitable protecting groups are t-butyloxycarbonyl (Boc), benzyloxycarbonyl (Cbz), biphenylisopropyloxycarbonyl, t-amyloxycarbonyl, isobornyloxycarbonyl, α, α-dimethyl-3,5-dimethoxybenzyloxycarbonyl, o-nitrophenylsulfenyl, 2-cyano-t-butyloxycarbonyl, 9-fluorenylmethyloxycarbonyl and the like, especially t-butyloxycarbonyl (Boc).

Particularly preferred side chain protecting groups are, for arginine: nitro, ptoluenesulfonyl, 4-methoxybenzenesulfonyl, Cbz, Boc and adamantyloxycarbonyl; for tyrosine: benzyl, o-bromobenzyloxycarbonyl, 2,6-dichlorobenzyl, isopropyl, cyclohexyl, cyclopentyl and acetyl; for serine: benzyl and tetrahydropyranyl; for histidine: benzyl, p-toluenesulfonyl and 2,4-dinitrophenyl.

The C-terminal amino acid is attached to a suitable solid support. Suitable solid supports useful for the above synthesis are those materials which are inert to the reagents and reaction conditions of the stepwise condensation-deprotection reactions, as well as being insoluble in the media used. Suitable solid supports are chloromethylpolystyrene-divinylbenzene polymer, hydroxymethyl-polystyrene-divinylbenzene polymer, and the like, especially chloromethyl-polystyrene-1% divinylbenzene polymer. For the special case where the C-terminus of the compound will be glycinamide, a particularly useful support is the benzhydrylamino-polystyrene-divinylbenzene polymer described by P. Rivaille, et al, Helv. Chim. Acta., 54, 2772 (1971). The attachment to the chloromethyl polystyrene-divinylbenzene type of resin is made by means of the reaction of the $N^{\alpha}$-protected amino acid, especially the Boc-amino acid, as its cesium, tetramethylammonium, triethylammonium, 1,5-diazabicyclo[5.4.0]undec-5-ene, or similar salt in ethanol, acetonitrile, N,N-dimethylformamide (DMF), and the like, especially the cesium salt in DMF, with the chloromethyl resin at an elevated temperature, for example between about 40 and 60° C, preferably about 50° C, for from about 12 to 48 hours, preferably about 24 hours. The $N^{\alpha}$-Boc-amino acid is attached to the benzhydrylamine resin by means of an N,N′-diisopropylcarbodiimide (DIC)/1-hydroxybenzotriazole (HBT) mediated coupling for from about 2 to about 24 hours, preferably about 12 hours at a temperature of between about 10 and 50° C, preferably 25° C in a solvent such as dichloromethane or DMF, preferably dichloromethane. The coupling of successive protected amino acids can be carried out in an automatic polypeptide synthesizer as is well known in the art. The removal

of the $N^{\alpha}$-protecting groups may be performed in the presence of, for example, a solution of trifluoroacetic acid in methylene chloride, hydrogen chloride in dioxane, hydrogen chloride in acetic acid, or other strong acid solution, preferably 50% trifluoroacetic acid in dichloromethane at about ambient temperature. Each protected amino acid is preferably introduced in approximately 2.5 molar excess and the coupling may be carried out in dichloromethane, dichloromethane/DMF mixtures, DMF and the like, especially in methylene chloride at about ambient temperature. The coupling agent is normally DCC in dichloromethane but may be N,N'-di-iso-propylcarbodiimide (DIC) or other carbodiimide either alone or in the presence of HBT, N-hydroxysuccinimide, other N-hydroxyimides or oximes. Alternately, protected amino acid active esters (e.g. p-nitrophenyl, pentafluorophenyl and the like) or symmetrical anhydrides may be used.

At the end of the solid phase synthesis the fully protected polypeptide is removed from the resin. When the linkage to the resin support is of the benzyl ester type, cleavage is by means of aminolysis with an alkylamine or fluoroalkylamine for peptides with a proline C-terminus, or by aminolysis with, for example, ammonia/methanol or ammonia/ethanol for peptides with a glycine C-terminus at a temperature between about 10 and 50° C, preferably about 25° C, for between about 12 and 24 hours preferably about 18 hours. Alternatively, the peptide may be removed from the resin by transesterification, e.g., with methanol, followed by aminolysis. The protected peptide may be purified at this point by silica gel chromatography. The removal of the side chain protecting groups from the polypeptide is performed by treating the aminolysis product with, for example, anhydrous liquid hydrogen fluoride in the presence of anisole or other carbonium scavenger, treatment with hydrogen fluoride/pyridine complex, treatment with tris(trifluoroacetyl)boron and trifluoroacetic acid, by reduction with hydrogen and palladium on carbon or polyvinylpyrrolidone, or by reduction with sodium in liquid ammonia, preferably with liquid hydrogen fluoride, and anisole at a temperature between about -10 and +10° C, preferably about 0° C, for between about 15 minutes and 1 hour, preferably about 30 minutes. For the glycine terminal peptides on the benzyhydrylamine resins, the resin cleavage and deprotection steps may be combined in a single step utilizing liquid hydrogen fluoride and anisole as described above. The fully deprotected polypeptide is then purified by a sequence of chromatographic steps employing any or all of the following types: ion exchange on a weakly basic resin in the acetate form; hydrophobic adsorption chromatography on underivatized polystyrene-divinylbenzene (for example Amberlite XAD); silica gel adsorption chromatography; ion exchange chromatography on carboxymethylcellulose; partition chromatography, e.g., on Sephadex G-25, or countercurrent distribution; high performance liquid chromatography (HPLC), especially reverse phase HPLC on octyl- or octadecylsilyl-silica bonded phase column packing.

If an racemic amino acid is used in the 1, 2, 3 or 6 position, the diastereomeric nonapeptide or decapeptide final products are separated, and the desired peptide containing a D-amino acid in the appropriate position is isolated and purified, preferably during the above-described chromatographic process.

The preparation of peptides having C-terminal azaglycine amides is preferably done using classical peptide solution synthesis using known peptide intermediates.

Thus, in general, the LHRH antagonists of the present invention may be prepared by a process comprises: removing protecting groups and, optionally, covalently bound solid support from a protected polypeptide to afford a compound of Formula (I) or a salt thereof, and optionally

(a) converting a compound of Formula (I) to a pharmaceutically acceptable salt, or

(b) converting a salt of a compound of Formula (I) to a pharmaceutically acceptable salt, or

(c) decomposing a salt of a compound of Formula (I) to a free polypeptide of Formula (I).

The following examples are given to enable those skilled in the art to more fully understand and practice the present invention. They should not be construed as a limitation upon the scope of the invention, but merely as being illustrative and representative thereof.

## Preparation A

### 3-(2-Naphthyl)-D,L-Alanine

The preparation of 3-(2-naphthyl)-D,L-alanine is carried out according to the procedure set out in U.S. Patent 4,341,767.

Preparation of N-acetyl-3-(2-naphthyl)-D,L-alinine, its conversion to methyl N-acetyl-3-(2-naphthyl)-D,L-alaninate, and separation of the D isomer is carried out by the procedure disclosed in U.S. Patent 4,341,767.

## Preparation B

Benzyl $N^{\alpha}$-benzyloxycarbonyl-N,N'-guanidino-diisopropyl-D-homoargininate toluenesulfonate

A mixture of 5.24 g of benzyl $N^{\alpha}$-benzyloxycarbonyl-D-lysinate toluenesulfonate (B. Bezus and L. Zervas, J. Am. Chem. Soc. 83 719 (1961)) and 1.72 ml of diisopropylethylamine in 60 ml of dioxane is treated with 1.89 g of N,N'-diisopropylcarbodiimide. The reaction mixture is stirred at 100° C for 6 hours, cooled to room temperature and concentrated to a solid. The solid is suspended in 20 ml of warm DMF, filtered to remove N,N'-diisopropylurea and the filtrate concentrated to a solid. Benzyl $N^{\alpha}$-benzyloxycarbonyl-N,N'-guanidino-diisopropyl-D-homoargininate toluenesulfonate is obtained as a white solid by crystallization from methanol/ethyl acetate $[\alpha]_D$ -7.26° (C 0.3, MeOH).

Similarly, by using the above procedure, but substituting:
N,N'-dicyclohexylcarbodiimide;
N,N'-di-n-hexylcarbodiimide;
N,N'-diethylcarbodiimide;
N,N'-di-n-propylcarbodiimide;
N-i-propylcarbodiimide;
N-propylcarbodiimide;
N-n-butylcarbodiimide;
N,N'-di-n-butylcarbodiimide;
N,N'-dimethylcarbodiimide;
N,N'-di-i-butylcarbodiimide;
N,N'-di-n-pentylcarbodiimide;
N,N'-di-i-pentylcarbodiimide;
N,N'-diphenylcarbodiimide;
N,N'-ditolylcarbodiimide; or
1-(3-dimethylaminopropyl)-3-ethylcarbodiimide-HCl; and the like, there are obtained:
benzyl $N^{\alpha}$-benzyloxycarbonyl-N,N'-guanidino-dicyclohexyl-D-homoargininate, $[\alpha]_D$ 8.07° (C 0.9 MeOH);
benzyl $N^{\alpha}$-benzyloxycarbonyl-N,N'-guanidino-diethyl-D-homoargininate;
benzyl $N^{\alpha}$-benzyloxycarbonyl-N,N'-guanidino-di-n-propyl-D-homoargininate $[\alpha]_D$ 8.07° (C 0.9 MeOH);
benzyl $N^{\alpha}$-benzyloxycarbonyl-N-guanidino-n-propyl-D-homoargininate;
benzyl $N^{\alpha}$-benzyloxycarbonyl-N-guanidino-n-butyl-D-homoargininate;
benzyl $N^{\alpha}$-benzyloxycarbonyl-N,N'-guanidino-di-n-butyl-D-homoargininate;
benzyl $N^{\alpha}$-benzyloxycarbonyl-N,N'-guanidino-di-i-butyl-D-homoargininate;
benzyl $N^{\alpha}$-benzyloxycarbonyl-N,N'-guanidino-di-n-pentyl-D-homoargininate;
benzyl $N^{\alpha}$-benzyloxycarbonyl-N,N'-guanidino-di-phenyl-D-homoargininate;
benzyl $N^{\alpha}$-benzyloxycarbonyl-N,N'-guanidino-dimethyl-D-homoargininate;
benzyl $N^{\alpha}$-benzyloxycarbonyl-N,N'-guanidino-di-n-hexyl-D-homoargininate; and
benzyl $N^{\alpha}$-benzyloxycarbonyl-N,N'-guanidino-di-isopropyl-D-argininate, $[\alpha]_D$ -10.5° (C 0.5, MeOH); as their benzenesulfonate salts. Similarly, by substituting benzyl $N^{\alpha}$-benzyloxycarbonyl-D-ornithinate for the D-lysinate there may be obtained the corresponding arginine analogs as their toluenesulfonate salts.

## Preparation C

Benzyl $N^{\alpha}$-benzyloxycarbonyl-N, N' -guanidinodiethyl-D-homoargininate

To a mixture of 15 ml of toluene and 15 ml of t-BuOH was added 2.71g of benzyl $N^{\alpha}$-benzyloxycarbonyl-lysinate and 1.46 g of 2-methylthioimidazoline·HI (available from Aldrich). The pH of the mixture was brought to about 8 by the addition of diisopropylethylamine and the solution was heated under reflux for 24 hours.

The solution was concentrated in vacuo and the residue was loaded on a silica gel column (250 g). The column was eluted with a gradient from $CH_2Cl_2$/MeOH (19:1) to $CH_2Cl_2$/MeOH (7.3). The fractions containing product were detected by TLC, pooled, and concentrated to dryness, 2.9 g of white foam.

A 2 g portion of the above-named product was dissolved in 50 ml of EtOH containing 0.8g of 10% Pd/C. The solution was stirred under $H_2$ for 8 hours. The mixture was filtered on celite and the filtrate was concentrated to dryness to give N, N'-guanidinodiethyl-homoarginine as a white foam, 1.2 g.

In a similar manner, but employing S-methyl 3,4,5,6-tetrahydro-2-pyrimidinethiol (Aldrich) as the guanylating species, was obtained N,N'-guanidino-dipropylhomoarginine as a white foam.

In a similar manner, but employing S-methyl bis-(2,2,2-trifluoroethyl)-thiouronium iodide as the guanylating species, there was obtained N,N'-bis(trifluoroethyl)-homoarginine (Bth) as a white foam.

## Preparation D

### $N^{\alpha}$-t-butyloxycarbonyl-N,N′-guanidino-diisopropyl-D-homoarginine toluenesulfonate

This Preparation illustrates the preparation of $N^{\alpha}$-t-butyloxycarbonyl derivatives of N,N′-guanidino-disubstituted-D-homoarginines from their toluenesulfonate precursors.

A mixture of benzyl $N^{\alpha}$-benzyloxycarbonyl-N,N′-guanidino-diisopropyl-D-homoargininate toluenesulfonate (3.25 g) and 100 mg of 10% Pd/C in 50 ml of glacial acetic acid is treated with hydrogen gas at atmospheric pressure for 4 hours. The catalyst is filtered on celite and the filtrate is concentrated to a solid, N,N′-guanidino-diisopropyl-D-homoarginine toluenesulfonate. A solution of this compound (2.13 g) in 60 ml of 50% dioxane/water is treated with 10 ml of 1N sodium hydroxide and 0.4 g of magnesium oxide. This mixture is then treated with 1.1 g of di-t-butyldicarbonate and stirred at room temperature for 2 hours. The magnesium salt is filtered and the filtrate is concentrated under vacuum. The basic solution is washed with ethanol, then brought to pH 2.5 with sodium sulfate. The acidic aqueous solution is extracted with ethylacetate which is dried over magnesium sulfate. The drying agent is filtered and the filtrate is concentrated. Crystallization from ethyl acetate/hexane affords $N^{\alpha}$-t-butyloxycarbonyl,N,N′-guanidino-diisopropyl-D-homoarginine toluenesulfonate.

Proceeding in a similar manner, but substituting the appropriate toluenesulfonate precursors, other $N^{\alpha}$-t-butyloxycarbonyl-N,N′-guanidino-disubstituted-D- homoarginine or D-arginine compounds may be prepared.

## PREPARATION E

### $N^{\alpha}$-t-butyloxycarbonyl-3-(4′-(1′-propylpiperidyl))-D-alanine

A 4.6 g portion of sodium metal was added to 400 ml of absolute ethanol and heated. To the resultant solution of sodium ethoxide was added 21.7 g of diethyl acetamidomalonate and 16.4 g of 4-picolyl chloride hydrochloride (Aldrich Chem. Co.). The reaction mixture was heated to 100° C for 4 hours, cooled, filtered and concentrated in vacuo. The mixture was loaded on a silica gel column in methylene chloride/methanol (19:1) and eluted with the same mixture. The product was located as a fast running UV positive spot by TLC on silica gel in methylene chloride/methanol (19:1). Combined fractions were concentrated to provide the product.

The product from the foregoing paragraph was dissolved in 200 ml of ethanol and treated with a solution of 2.72 g of sodium hydroxide in 40 ml of water at 50° C for 6 hours. The solution was acidified with 12 ml of 6N HCl, concentrated to dryness and taken up in 200 ml of dioxane. The suspension was filtered and the filtrate heated at reflux for 2 hours. The solution was cooled and concentrated to dryness to yield ethyl $N^{\alpha}$-acetyl-3-(4-pyridyl)-D,L-alanine as a white solid.

A portion of this N-acetyl ester was resolved by treatment with 200 ml of the enzyme subtilisin Carlsberg (Sigma Chem. Co., protease VIII) in a mixture of 300 ml of dimethyl sulfoxide and 400 ml of 0.01M KCl (pH 7.2). The pH was maintained by addition of 1N NaOH on a pH stat. After a 6 hour period, the resolution was complete. The solution was diluted with 400 ml of water and extracted with 4 X 750 ml of ethyl acetate. The organic layers were combined and dried over magnesium sulfate and concentrated to yield ethyl $N^{\alpha}$-acetyl-3-(4-pyridyl)-D-alaninate as an oil.

The oil was reacted with 1.22 g of n-propyl bromide in 50 ml of ethanol after which the solution was concentrated to dryness to yield ethyl $N^{\alpha}$-acetyl-3-(1-propyl-pyridinium-4-yl)-D-alaninate bromide as a white hygroscopic solid.

This white solid was dissolved in 200 ml of ethanol and was reduced under an atmosphere of hydrogen gas using 100 mg of 10% Pd/C as a catalyst. After an 18 hour reduction period, the catalyst was filtered out and the solution concentrated to yield ethyl $N^{\alpha}$-acetyl-3-(4′-(1′-propylpiperidyl))-D-alaninate as a tan solid. The free acid was prepared by refluxing the ethyl ester in 100 ml of 6N HCl for 4 hours to yield 3-(4′-(1′-propylpiperidyl))-D-alanine as a white solid.

The free acid was dissolved in 100 ml of dioxane/water (1:1) and treated with 2 g of di-t-butyldicarbonate. The pH was maintained at 9 by addition of 1N NaOH on a pH stat. After 2 hours the reaction mixture was concentrated in vacuo, washed with 100 ml of ethyl ether and the aqueous layer was loaded on an Amberlite XAD-2 hydrophobic resin. The column was eluted with 250 ml of water followed by 250 ml of 50% ethanol/water. The ethanol eluate was pooled and concentrated to dryness to yield $N^{\alpha}$-t-butyloxycarbonyl--3-(4′-(1′-propylpiperidyl))-D-alanine as a white solid.

Proceeding in similar manner, but substituting 3-picolyl chloride hydrochloride for 4-picolyl chloride hydrochloride, there is prepared $N^{\alpha}$-t-butyloxycarbonyl-3-(3′-(1′-propylpiperidyl))-D-alanine.

## Preparation F

## Boc-Gly-O-Resin

4.9 g of Boc-glycine was dissolved in a mixture of 50 ml. ethanol and 50 ml. distilled water. The pH of the solution was brought to 7 with aqueous cesium bicarbonate. The solvent was then removed under vacuum. After 18 hours of drying under high vacuum, the residue was dissolved in 150 ml. dry DMF. 25 g chloromethylated polystyrene - 1% divinylbenzene (Merrifield) resin (corresponding to 25 mmole chloride) was added. The mixture was shaken at 50° C for 24 hours, filtered, and the resin was then washed sequentially with DMF, water, and ethanol. The resin was dried under vacuum for 3 days to yield 28.34 g of Boc-Gly-O-Resin.

## Preparation G

### A. S-Methyl 3,4,5,6-tetrahydro-2-pyrimidine thio ·hydroiodide

A solution of 23.24 g of 3,4,5,6-tetrahydro-2-pyrimidine thiol in 175 ml of MeOH was treated with 15.57 ml of MeI and refluxed for 1.5 hr. The solvent was evaporated under vacuum and the residue suspended in $Et_2O$. The precipitate was filtered and dried in vacuo to provide S-Methyl 3,4,5,6-tetrahydro-2-pyrimidine thiol as 51.4 g of white crystals.

### B. $N^{\alpha}$-t-Butyloxycarbonyl-$N^{G}$, $N^{G'}$ -propano-L-homoarginine

S-Methyl 3,4,5,6-tetrahydro-2-pyrimidine thiol was sprung from 51.4 g of its HI salt by partitioning between 300 ml of $CH_2Cl_2$ and 50 ml of 4N NaOH. The resulting $CH_2Cl_2$ solution was evaporated to ~100 ml and ~100 ml of EtOH was added. A solution of 24 g of lysine hydrochloride in 66 ml of 2N NaOH was treated at 60 C in a dropwise fashion with the solution of S-methyl 3,4,5,6-tetrahydro-2-pyrimidine thiol. Stirring was continued overnight at 60 °C under $N_2$. An additional 10.78 g of the HI salt was sprung with 15 ml of 4N NaOH, extracted with 90% of $CH_2Cl_2$ and added dropwise with stirring for another 24 hour period at 60 C at which time the reaction was essentially complete.

The reaction mixture was washed with 2 portions of EtOAc and the aqueous layer containing Pha was diluted with 200 ml of dioxane and 200 ml of $H_2O$. The solution was cooled to 0 °C before addition of 33 g of di-t-butyldicarbonate and 6 g of MgO. An additional batch of 4 g of MgO and 22 g of di-t-butyldicarbonate pushed the reaction to completion.

The MgO was filtered on celite and the filtrate was evaporated in vacuo to 1/2 volume. The residue was washed twice with $Et_2O$ before loading on a silica gel column (750 g silica gel packed in $CH_3CN$). The column was washed with 5L $CH_3CN$, eluted with 10% $H_2O/CH_3CN$ (2L), and further eluted with 20% $H_2O/CH_3CN$ (5L). The product fractions were located by thin layer chromatography ($CH_3CN/HOAc/H_2O$; 4:1:1) on silica gel plates. The product fractions were pooled and concentrated to yield the product as 5.04 g of white foam of m.p. 96 °C, $[\alpha]_D^{25}$ 16.1 °C (C 0.54, MeOH). and an additional 15 g of slightly impure oil.

In a similar fashion but substituting the appropriate S-Methyl N,N′-dialkylthiouronium hydroiodides are obtained the corresponding $N^{\alpha}$-t-butyloxycarbonyl-$N^{G}$, $N^{G'}$ -dialkylhomoarginines.

## Example 1

N-Ac-D-Nal(2)-D-pCl-Phe-D-Pal(3)-Ser-hArg($CH_2CF_3$)$_2$-D-Tyr-Leu-hArg($CH_2CF_3$)$_2$-Pro-D-Ala-$NH_2$

The title compound is also represented herein as N-Ac-D-Nal(2)-D-pCl-Phe-D-Pal(3)-Ser-Bth-D-Tyr-Leu-Bth-Pro-D-Ala-$NH_2$.

In the reaction vessel of a Beckman 990 Peptide Synthesizer was placed 0.758 g. (0.5 mmol.) of benzhydrylamino-polystyrene resin (Peninsula Labs, 0.66 mmol/g). The first amino acid was coupled by addition of 0.284 g of Boc-D-Ala-OH, 0.202 g of HBt and 3 ml of 0.5 M diisopropylcarbodiimide. After a 3 hour coupling period and washing of the resin, further amino acids were added sequentially to this resin by means of a synthesis program, as follows:

| | | | |
|---|---|---|---|
| Step 1 | $CH_2Cl_2$ wash | 1 time | 1.5 min |
| 2 | 50% $CF_3CO_2H/CH_2Cl_2$--deprotection | 1 time | 1.5 min |
| 3 | 50% $CF_3CO_2H/CH_2Cl_2$--deprotection | 1 time | 30 min |
| 4 | $CH_2Cl_2$ wash | 3 times | 1.5 min |
| 5 | 10% triethylamine/$CH_2Cl_2$ | 2 times | 1.5 min |
| 6 | $CH_2Cl_2$ wash | 3 times | 1.5 min |
| 7 | $N^{\alpha}$-Boc-amino acid solution in 50% $CH_2Cl_2$/DMF | 1 time | add |
| 8 | N,N'-diisopropylcarbodiimide solution (0.5 M) | 1 time | add |
| 9 | $CH_2Cl_2$ rinse and hold--coupling | 1 time | coupling reaction 2 hr |
| 10 | $CH_2Cl_2$--rinse add | 1 time | 1.5 min |
| 11 | $CH_2Cl_2$ wash | 3 times | 1.5 min |
| 12 | ethanol wash | 3 times | 1.5 min |
| 13 | $CH_2Cl_2$ wash | 3 times | 1.5 min |

Steps 1-13 complete a coupling cycle for one amino acid and completeness of the reaction may be checked by the ninhydrin method of E. Kaiser, et al., Anal. Biochem., 34, 595 (1970).

The resin was coupled sequentially with a 2.0 to 3.0 molar excess of each protected amino acid and DIC. Thus, the resin was treated during successive coupling cycles with
0.269 g. Boc-Pro-OH,
0.610 g. Boc-Bth-OH,
0.311 g. Boc-Leu-OH·$H_2O$
0.375 g. Boc-D-Tyr-OH,
0.610 g. Boc-Bth-OH,
0.380 g. Boc-Ser(Benzyl)-OH,
0.320 g. Boc-D-Pal(3)-OH,
0.390 g. Boc-D-pCl-Phe-OH,
0.400 g. Boc-D-Nal(2)-OH, and
2.5 ml. acetic anhydride.

The resin was removed from the reaction vessel, washed with $CH_2Cl_2$, and dried in vacuo to yield 1.43 g. of protected polypeptide resin. The protected peptide was deprotected and removed from the resin by treatment with 25 ml. anhydrous liquid HF in the presence of 2.5 ml. of anisole (scavenger) in a Kel-F reaction vessel at

0° C for 1 hr. The HF was evaporated under vacuum and the residue of N-Ac-D-Nal(2)-D-pCl-Phe-D-Pal(3)-Ser-hArg($CH_2CF_3$)$_2$-D-Tyr-Leu-hArg($CH_2CF_3$)$_2$-Pro-D-Ala-$NH_2$, as its HF salt, was washed with ether. The residue was then extracted with glacial acetic acid (3 x 30 ml). The acetic acid extract was evaporated to dryness. The crude material was converted to the acetate salt by passage in water through a column of AG3 (a weakly basic tertiary amine resin) which had been converted to the acetate form. Lyophilization of the eluate yielded 0.5 g. of the crude peptide acetate salt as a white solid.

The crude peptide was purified by high performance liquid chromatography on a 2.5 x 100 cm. column of Licroprep RP-18 (25-40 micron) equilibrated to the running buffer 50% $CH_3CN$/ 50% $H_2O$ (0.1% in $CF_3CO_2H$, pH 2.5). The major UV absorbing (280 nm) peak eluting at approximately 2 column volumes was collected, concentrated to dryness, and lyophilized 3 times from distilled water to yield 64 mg of pure N-Ac-D-Nal(2)-D-pCl-Phe-D-Pal(3)-Ser-hArg($CH_2CF_3$)$_2$-D-Tyr-Leu-hArg($CH_2CF_3$)$_2$-Pro-D-Ala-$NH_2$, $[\alpha]_D^{25}$ = -17.96° (C 0.4, HOAc).

B. Proceeding in a similar manner but substituting the appropriate a, b, c, d, e, f, g, h or j amino acid for those recited, there were prepared the corresponding decapeptides exemplified below:

N-Ac-D-Nal(2)-D-pCl-Phe-D-Pal(3)-Ser-Tyr-D-Pal(3)-Leu-Deh-Pro-D-Ala$NH_2$;
N-Ac-D-Nal(2)-D-pCl-Phe-D-Pal(3)-Ser-Mbh-D-Pal(3)-Leu-Mbh-Pro-D-Ala$NH_2$;
N-Ac-D-Nal(2)-D-pCl-Phe-D-Pal(3)-Ser-Pal(3)-D-Pal(3)-Leu-Bth-Pro-D-Ala$NH_2$;
N-Ac-D-Nal(2)-D-pCl-Phe-D-Pal(3)-Ser-Arg-D-Pal(3)-Leu-Deh-Pro-D-Ala$NH_2$;
N-Ac-D-Nal(2)-D-pCl-Phe-D-Pal(3)-Ser-Tyr-D-Pal(3)-Leu-Bth-Pro-D-Ala$NH_2$;
N-Ac-D-Nal(2)-D-pCl-Phe-D-Pal(3)-Ser-Tyr-D-Pal(3)-Leu-Mbh-Pro-D-Ala$NH_2$;
N-Ac-D-Nal(2)-D-pCl-Phe-D-Pal(3)-Ser-Tyr-D-Pal(3)-Leu-Pha-Pro-D-Ala$NH_2$;
N-Ac-D-Nal(2)-D-pCl-Phe-D-Trp-Ser-Tyr-D-Trp-Leu-Deh-Pro-D-Ala$NH_2$;
N-Ac-D-Nal(2)-D-pCl-Phe-D-Trp-Ser-Arg-D-Trp-Leu-Deh-Pro-D-Ala$NH_2$;
N-Ac-D-Nal(2)-D-pCl-Phe-D-Trp-Ser-Arg-D-Trp-Leu-Bth-Pro-D-Ala$NH_2$;
N-Ac-D-Nal(2)-D-pCl-Phe-D-Trp-Ser-Arg-D-Trp-Leu-Pha-Pro-D-Ala$NH_2$;
N-Ac-D-Nal(2)-D-pCl-Phe-D-Pal(3)-Ser-Arg-D-Pal(3)-Leu-Pha-Pro-D-Ala$NH_2$;
N-Ac-D-Nal(2)-D-pCl-Phe-D-Trp-Ser-Tyr-D-Bth-Leu-Bth-Pro-D-Ala$NH_2$;
N-Ac-D-Nal(2)-D-pCl-Phe-D-Trp-Ser-Tyr-D-Pha-Leu-Pha-Pro-D-Ala$NH_2$;
N-Ac-D-Nal(2)-D-pCl-Phe-D-Pal(3)-Ser-Tyr-D-Deh-Leu-Deh-Pro-D-Ala$NH_2$; and
N-Ac-D-Nal(2)-D-pCl-Phe-D-Pal(3)-Ser-Tyr-D-Bth-Leu-Bth-Pro-D-Ala$NH_2$.

C. Proceeding in a similar manner but substituting the appropriate a, b, c, d, e, f, g, h or j amino acid for those recited, there are prepared the corresponding decapeptides exemplified below:

N-Ac-D-Nal(2)-D-pCl-Phe-D-Trp-Ser-Tyr-D-Trp-Leu-Deh-Pro-D-Ala$NH_2$;
N-Ac-D-Nal(2)-D-pCl-Phe-D-Trp-Ser-Tyr-D-Trp-Leu-Mbh-Pro-D-Ala$NH_2$;
N-Ac-D-Nal(2)-D-pCl-Phe-D-Trp-Ser-Tyr-D-Trp-Leu-Pha-Pro-D-Ala$NH_2$;
N-Ac-D-Nal(2)-D-pCl-Phe-D-Trp-Ser-Tyr-D-Deh-Leu-Deh-Pro-D-Ala$NH_2$;
N-Ac-D-Nal(2)-D-pCl-Phe-D-Trp-Ser-Tyr-D-Mbh-Leu-Mbh-Pro-D-Ala$NH_2$;
N-Ac-D-Nal(2)-D-pCl-Phe-D-Pal(3)-Ser-Tyr-D-Mbh-Leu-Mbh-Pro-D-Ala$NH_2$;
N-Ac-D-Nal(2)-D-pCl-Phe-D-Pal(3)-Ser-Tyr-D-Pha-Leu-Pha-Pro-D-Ala$NH_2$;
N-Ac-D-Nal(2)-D-pCl-Phe-D-Trp-Ser-Arg-D-Trp-Leu-Mbh-Pro-D-Ala$NH_2$;
N-Ac-D-Nal(2)-D-pCl-Phe-D-Pal(3)-Ser-Arg-D-Pal(3)-Leu-Bth-Pro-D-Ala$NH_2$;
N-Ac-D-Nal(2)-D-pCl-Phe-D-Pal(3)-Ser-Arg-D-Pal(3)-Leu-Mbh-Pro-D-Ala$NH_2$;
N-Ac-D-Nal(2)-D-pCl-Phe-D-Trp-Ser-Arg-D-Tyr-Leu-Deh-Pro-D-Ala$NH_2$;
N-Ac-D-Nal(2)-D-pCl-Phe-D-Trp-Ser-Arg-D-Tyr-Leu-Bth-Pro-D-Ala$NH_2$;
N-Ac-D-Nal(2)-D-pCl-Phe-D-Trp-Ser-Arg-D-Tyr-Leu-Mbh-Pro-D-Ala$NH_2$;
N-Ac-D-Nal(2)-D-pCl-Phe-D-Trp-Ser-Arg-D-Tyr-Leu-Pha-Pro-D-Ala$NH_2$;
N-Ac-D-Nal(2)-D-pCl-Phe-D-Pal(3)-Ser-Arg-D-Tyr-Leu-Bth-Pro-D-Ala$NH_2$;
N-Ac-D-Nal(2)-D-pCl-Phe-D-Pal(3)-Ser-Arg-D-Tyr-Leu-Mbh-Pro-D-Ala$NH_2$;
N-Ac-D-Nal(2)-D-pCl-Phe-D-Pal(3)-Ser-Arg-D-Tyr-Leu-Deh-Pro-D-Ala$NH_2$;
N-Ac-D-Nal(2)-D-pCl-Phe-D-Pal(3)-Ser-Arg-D-Tyr-Leu-Pha-Pro-D-Ala$NH_2$;
N-Ac-D-Nal(2)-D-pCl-Phe-D-Trp-Ser-Deh-D-Tyr-Leu-Deh-Pro-D-Ala$NH_2$;
N-Ac-D-Nal(2)-D-pCl-Phe-D-Trp-Ser-Mbh-D-Tyr-Leu-Mbh-Pro-D-Ala$NH_2$;
N-Ac-D-Nal(2)-D-pCl-Phe-D-Trp-Ser-Bth-D-Tyr-Leu-Bth-Pro-D-Ala$NH_2$;
N-Ac-D-Nal(2)-D-pCl-Phe-D-Trp-Ser-Pha-D-Tyr-Leu-Pha-Pro-D-Ala$NH_2$;
N-Ac-D-Nal(2)-D-pCl-Phe-D-Trp-Ser-Deh-D-Trp-Leu-Deh-Pro-D-Ala$NH_2$;
N-Ac-D-Nal(2)-D-pCl-Phe-D-Trp-Ser-Mbh-D-Trp-Leu-Mbh-Pro-D-Ala$NH_2$;
N-Ac-D-Nal(2)-D-pCl-Phe-D-Trp-Ser-Bth-D-Trp-Leu-Bth-Pro-D-Ala$NH_2$;
N-Ac-D-Nal(2)-D-pCl-Phe-D-Trp-Ser-Pha-D-Trp-Leu-Pha-Pro-D-Ala$NH_2$;
N-Ac-D-Nal(2)-D-pCl-Phe-D-Pal(3)-Ser-Deh-D-Pal(3)-Leu-Deh-Pro-D-Ala$NH_2$;
N-Ac-D-Nal(2)-D-pCl-Phe-D-Pal(3)-Ser-Mbh-D-Pal(3)-Leu-Mbh-Pro-D-Ala$NH_2$;
N-Ac-D-Nal(2)-D-pCl-Phe-D-Pal(3)-Ser-Bth-D-Pal(3)-Leu-Bth-Pro-D-Ala$NH_2$;
N-Ac-D-Nal(2)-D-pCl-Phe-D-Pal(3)-Ser-Pha-D-Pal(3)-Leu-Pha-Pro-D-Ala$NH_2$;
N-Ac-D-Nal(2)-D-pCl-Phe-D-Trp-Ser-Deh-D-Nal(2)-Leu-Deh-Pro-D-Ala$NH_2$;
N-Ac-D-Nal(2)-D-pCl-Phe-D-Trp-Ser-Mbh-D-Nal(2)-Leu-Mbh-Pro-D-Ala$NH_2$;
N-Ac-D-Nal(2)-D-pCl-Phe-D-Trp-Ser-Bth-D-Nal(2)-Leu-Bth-Pro-D-Ala$NH_2$;

N-Ac-D-Nal(2)-D-pCl-Phe-D-Trp-Ser-Pha-D-Nal(2)-Leu-Pha-Pro-D-Ala$NH_2$;
N-Ac-D-Nal(2)-D-pCl-Phe-D-Pal(3)-Ser-Pal(3)-D-Pal(3)-Leu-Deh-Pro-D-Ala$NH_2$;
N-Ac-D-Nal(2)-D-pCl-Phe-D-Pal(3)-Ser-Pal(3)-D-Pal(3)-Leu-Bth-Pro-D-Ala$NH_2$;
N-Ac-D-Nal(2)-D-pCl-Phe-D-Pal(3)-Ser-Pal(3)-D-Pal(3)-Leu-Pha-Pro-D-Ala$NH_2$; and
N-Ac-D-Nal(2)-D-pCl-Phe-D-Pal(3)-Ser-Pal(3)-D-Pal(3)-Leu-Mbh-Pro-D-Ala$NH_2$.

## Example 2

### N-Ac-D-Nal(2)-D-pCl-Phe-D-Pal(3)-Ser-hArg($CH_2CF_3$)$_2$-D-Tyr-Leu-hArg($CH_2CF_3$)$_2$-Pro-NHEt

For the synthesis of analogs with a C-terminal Pro-$NHCH_2CH_3$, a synthesis program identical to that described in Example 1 is used. The Beckman 990 Synthesizer reaction vessel is loaded with 0.71 g. of Boc-Pro-O-Resin, prepared by the reaction of 1.3 molar excess of the dry cesium salt of Boc-Pro-OH with chloromethyl-polystyrene/1% divinylbenzene (Lab Systems, Inc.). The quantity of Boc-Pro-O-Resin taken contains 0.5 mmol. of proline.

The resin is coupled sequentially with a 2.0 to 3.0 molar excess of each protected amino acid and DIC. Thus, the resin is reacted during successive coupling cycles with
0.610 g. Boc-Bth-OH,
0.311 g. Boc-Leu-OH·$H_2O$
0.375 g. Boc-D-Tyr-OH,
0.610 g. Boc-Bth-OH,
0.380 g. Boc-Ser(Benzyl)-OH,
0.320 g. Boc-D-Pal(3)-OH,
0.390 g. Boc-D-pCl-Phe-OH,
0.400 g. Boc-D-Nal(2)-OH, and
2.5 ml. acetic anhydride.

The resin is removed from the reaction vessel, washed with $CH_2Cl_2$, and dried in vacuo to yield 1.5 g. of protected polypeptide resin.

The protected polypeptide is cleaved from the resin by aminolysis with 25 ml. of ethylamine for 18 hours at 2°C. The ethylamine is allowed to evaporate and the resin is extracted with methanol. The methanol is evaporated to yield 0.7 g. of protected peptide. This protected peptide is mixed with 2.5 ml of anisole and 25 ml of redistilled (from $CoF_3$) anhydrous liquid HF at 0°C. for 1 hour in a Kel-F reaction vessel. The HF is evaporated under vacuum and the residue is washed with ether. The residue is dissolved in 2 M acetic acid and converted to the acetate salt by passage in water through a column of AG3 which had been converted to the acetate form. Lyophilization of the eluate yields 0.5 g. of the crude peptide acetate salt as a white solid. Final purification is achieved by preparative high performance liquid chromatography on a 2.5 x 100 mm. column of 40-50 micron octadecylsilylated silica (Merck, Lichroprep $C_{18}$) using 50% $CH_3OH$ (0.1% $CF_3CO_2H$) as eluate. Lyophilization of the product from $H_2O$ yields N-Ac-D-Nal(2)-D-pCl-Phe-D-Pal(3)-Ser-hArg($CH_2CF_3$)$_2$-D-Tyr-Leu-hArg($CH_2CF_3$)$_2$-ProNHEt as 70 mg of white powder.

Proceeding in a similar manner, but substituting the required protected amino acid residues where appropriate, there are prepared the following compounds:
N-Ac-D-Nal(2)-D-pCl-Phe-D-Pal(3)-Ser-Tyr-D-Pal(3)-Leu-Bth-Pro-NHEt,
N-Ac-D-Nal(2)-D-pCl-Phe-D-Pal(3)-Ser-Tyr-D-Pal(3)-Leu-Mbh-Pro-NHEt,
N-Ac-D-Nal(2)-D-pCl-Phe-D-Pal(3)-Ser-Tyr-D-Pal(3)-Leu-Pha-Pro-NHEt,
N-Ac-D-Nal(2)-D-pCl-Phe-D-Pal(3)-Ser-Tyr-D-Pal(3)-Leu-Deh-Pro-NHEt,
N-Ac-D-Nal(2)-D-pCl-Phe-D-Trp-Ser-Arg-D-Nal(2)-Leu-Bth-Pro-NHEt

Repeating the above cleavage, substituting a stoichiometric amount of methylamine and propylamine for ethylamine there are obtained the corresponding methylamides or propylamides of the aforementioned nonapeptides.

## Example 3

### Preparation of Salts

A. A solution of 0.1 g of the hydrogen fluoride salt of (N-Ac-D-Nal(2)$^1$, D-pCl-Phe$^2$, D-Pal(3)$^{3,6}$, Bth$^8$,

D-Ala[10])LHRH (See Example 1) is dissolved in 50 ml of water and passed through a column of 50 g Dowex 3 anion exchange resin which had previously been equilibrated with acetic acid and washed with deionized water. The column is eluted with deionized water and the effluent is lyophilized to yield the corresponding acetic acid salt of (N-Ac-D-Nal(2)$^1$, D-pCl-Phe$^2$, D-Pal(3)$^{3,6}$, Bth$^8$, D-Ala$^{10}$)LHRH.

Repeating the above, substituting other acids for acetic acid during the equilibration of the resin, there may be obtained, for example, the corresponding salts with hydrochloric acid, hydrobromic acid, sulfuric acid, phosphoric acid, nitric acid, benzoic acid, and the like.

Similarly there may be prepared the acid addition salts of the other peptides analogous to LHRH, described herein.

B. In the case of salts of low water solubility, these may be prepared by precipitation from water utilizing the desired acid. For example:

Zinc tannate salt - a solution of 10 mg of (N-Ac-D-Nal(2)$^1$, D-pCl-Phe$^2$, D-Pal(3)$^{3,6}$, Bth$^8$, D-Ala$^{10}$)LHRH acetic acid salt in 0.1 ml of water was treated with a solution of 8 mg of tannic acid in 0.08 ml of 0.25 M NaOH. A solution of 5 mg of $ZnSO_4$ heptahydrate in 0.1 ml of water was immediately added to the solution of the LHRH analog.

The resultant suspension was diluted with 1 ml water and the precipitate was centrifuged. The supernatant was decanted and the residue was washed twice with 1 ml portions of water by centrifugation of the precipitate and decantation of the supernatant. The precipitate was dried in vacuo to yield 15 mg of the mixed zinc tannate salt of the above named LHRH analog.

Pamoate salt - to a solution of 50 mg (N-Ac-D-Nal(2)$^1$, D-pCl-Phe$^2$, D-Pal(3)$^{3,6}$, Bth$^8$, D-Ala$^{10}$)LHRH acetic acid salt in a mixture of 1.6 ml of ethanol and 0.1 ml of 0.25 M NaOH was added a solution of 11 mg of pamoic acid in 0.3 ml of 0.25 M NaOH. The solvents were removed at reduced pressure and the residue was suspended in 2 ml of water, centrifuged, and the supernatant was decanted. The precipitate was washed with 1.5 ml $H_2O$, centrifuged, and the supernatant was decanted. The precipitate was dried in vacuo to yield 54 mg of the pamoate salt of the above named LHRH analog.

In a similar manner other salts of low water solubility may be prepared.

C. Preparation of acid addition salt from free peptide.

To a solution of 50 mg of (N-Ac-D-Nal(2)$^1$, D-pCl-Phe$^2$, D-Pal(3)$^{3,6}$, Bth$^8$, D-Ala$^{10}$)LHRH as the free base is added 30 ml of 1N acetic acid. The resulting solution is lyophilized to yield 50 mg. of the acetic acid salt of the above.

Similarly, replacing acetic acid with other acids (in stoichiometrically equivalent amounts relative to peptide) there are obtained other acid addition salts of the peptides herein, for example, the salts with hydrochloric acid, hydrobromic acid, sulfuric acid, phosphoric acid, nitric acid.

D. Preparation of salt with metal cation, e.g., zinc salt.

To a solution of 50 mg (N-Ac-D-Nal(2)$^1$, D-pCl-Phe$^2$, D-Pal(3)$^{3,6}$, Bth$^8$, D-Ala$^{10}$)LHRH acetic acid salt in a mixture of 0.4 ml of 0.25 M NaOH, 0.3 ml water, and 1 ml ethanol was added a solution of 15 mg of $ZnSO_4$ heptahydrate in 0.2 ml of water. The precipitate was centrifuged and the supernatant was decanted. The precipitate was washed with 1 ml of water by centrifugation and decantation of the supernatant. The precipitate was dried in vacuo to yield the zinc salt of the above named LHRH analog.

In a similar manner salts with other multivalent cations e.g. calcium, bismuth, barium, magnesium, aluminum, copper, cobalt, nickel, cadmium and the like, may be prepared.

## Example 4

## Conversion of Salts to Free Base

A solution of 50 mg of (N-Ac-D-Nal(2)$^1$, D-pCl-Phe$^2$, D-Pal(3)$^{3,6}$, Bth$^8$, D-Ala$^{10}$)LHRH acetic acid salt in 25 ml. of water is passed through a 50 g column of Dowex 1 (strongly basic, quaternary ammonium anion exchange resin) which had been equilibrated with NaOH solution to make the counter ion hydroxide. The column is eluted with 150 ml of water and the eluant is lyophilized to yield 45 mg of the corresponding polypeptide as the free base.

Similarly other acid addition salts of compounds of the peptides herein, e.g., those mentioned in Example 6, may be converted to the corresponding free bases.

## Example 5

## Pharmaceutical Formulations

The following are typical pharmaceutical compositions containing, as active ingredient, an LHRH antagonist of the present invention, for example (N-Ac-D-Nal(2)$^1$, D-pCl-Phe$^2$, D-Pal(3)$^{3,6}$, Deh$^8$, D-Ala$^{10}$)LHRH, by itself or as a pharmaceutically acceptable salt, e.g., the acetic acid addition salt, the zinc salt, the zinc tannate salt, etc.

### A. Tablet Formulations

1. LHRH Antagonist 10.0 mg
Compressible Sugar, USP 86.0 mg
Calcium Stearate 4.0 mg
2. LHRH Antagonist 10.0 mg
Compressible Sugar, USP 88.5 mg
Magnesium Stearate 1.5 mg
3. LHRH Antagonist 5.0 mg
Mannitol, USP 83.5 mg
Magnesium Stearate, USP 1.5 mg
Pregelatinized Starch, USP 10.0 mg
4. LHRH Antagonist 10.0 mg
Lactose, USP 74.5 mg
Pregelatinized Starch, USP 15.0 mg
Magnesium Stearate, USP 1.5 mg

### Method of Manufacture

(a) LHRH Antagonist is dissolved in water, a sufficient quantity to form a wet granulation when mixed with the sugar portion of the excipients. After complete mixing, the granulation is dried in a tray or fluid-bed dryer. The dry granulation is then screened to break up any large aggregates and then mixed with the remaining components. The granulation is then compressed on a standard tabletting machine to the specific tablet weight.

(b) In this manufacturing method, all formulations would include 0.01% gelatin, USP. The gelatin would be first dissolved in the aqueous granulation solvent followed by the LHRH analog. The remaining steps are as in (a) above.

Formulation 4 could also be used as a tablet for oral administration.

The progestational agent can be optionally included in the formulation.

### B. Nasal Formulations

#### 1. Freeze Dried Powder Formulation

10 milligrams of LHRH antagonist and 240 mg of dextran T70 are dissolved together in purified water. A powdered formulation having 4 wt.% LHRH antagonist is then made by freeze drying the solution, grinding the product in a pestle and mortar and passing the resultant powder through a #200 standard mesh.

Similarly, by varying the proportion of LHRH analog to dextran T70 and following the procedure above, 2 wt% and 8 wt.% powdered formulations are prepared.

The progestational agent can be incorporated into this nasal formulation, or can be administered separately, in tablet form.

#### 2. Freeze Dried Powder Formulation containing an Absorption Enhancer

10 milligrams of LHRH antagonist, 235 mg of dextran T70 and 5 mg of sodium glycocholate are dissolved together in purified water. A powdered formulation having 4 wt.% LHRH antagonist and 2 wt.% of sodium glycocholate is then made by freeze drying the solution, grinding the product in a pestle and mortar and passing the resultant powder through a #200 standard mesh.

Similarly, by varying the proportion of LHRH analog to dextran T70 and following the procedure above, 2 wt% and 8 wt.% powdered formulations are prepared.

The progestational agent can be incorporated into this nasal formulation, or can be administered separately, in tablet form.

C. Long Acting Intramuscular Injectable Formulation

1. Long Acting I.M. Injectable - Sesame Oil Gel
LHRH Antagonist 100.0 mg
Optional 19-Nor Progestagen 30.0 mg
Aluminum monostearate, USP 20.0 mg
Sesame oil q.s. ad 1.0 ml

The aluminum monostearate is combined with the sesame oil and heated to 125°C with stirring until a clear yellow solution forms. This mixture is then autoclaved for sterility and allowed to cool. The LHRH analog is then added aseptically with trituration. Particularly preferred LHRH analogs are salts of low solubility, e.g. zinc salts, zinc tannate salts, pamoate salts, and the like. These exhibit exceptionally long duration of activity. The progestagen can be optionally included in the formulation.

2. Long Acting I.M. Injectable - Biodegradable Polymer Microcapsules

a. Without progestagen:
LHRH Antagonist 40%
25/75 glycolide/lactide copolymer (0.5 intrinsic viscosity) 60%

b. With progestagen:
LHRH Antagonist 40%
19-nor progestational agent 30%
25/75 glycolide/lactide copolymer (0.5 intrinsic viscosity) 30%
Microcapsules of above formulations suspended in:
Dextrose 5.0%
CMC, sodium 0.5%
Benzyl alcohol 0.9%
Tween 80 0.1%
Water, purified q.s. 100.0%
25 mg of microcapsules would be suspended in 1.0 ml of vehicle.

D. Aqueous Solution for Intramuscular Injection
Progestagen 0.4%
LHRH Antagonist 0.5%
Acetic Acid 0.02% M
Benzyl Alcohol 0.9%
Mannitol 3.5%
Propylene Glycol 20%
NaOH sufficient to adjust pH to 5
Water q.s. to 100%

Acetic acid, benzyl alcohol, mannitol and propylene glycol were dissolved in 90% of the water. Then the Antagonist was dissolved in this solution, and the pH adjusted with NaOH. Water was added to q.s. The solution was filtered through a one micron filter, packaged into vials, and sterilized by autoclaving.

E. Aqueous Formulation for Nasal Administration
Optional progestagen 35 mg
LHRH Antagonist 50 mg
0.02M Acetate Buffer 5 ml
Sodium Glycocholate 500 mg
0.02 Acetate Buffer, pH 5.2 q.s. 10ml

F. Formulation for Rectal Administration
Suppository Vehicle:
LHRH Antagonist 5.0 mg
Witepsol H15 20.0 gm

The LHRH antagonist is combined with the molten Witepsol H15, mixed well and poured into 2 gm molds.

**Claims**

1. A pharmaceutical composition, comprising an LHRH antagonist and a menopausal-symptom-alleviating amount of a 19-nor progestational agent arranged for simultaneous or sequential administration.

2. A pharmaceutical composition according to Claim 1, wherein the LHRH antagonist and the 19-nor progestational agent are in a single formulation.

3. The composition of Claim 1, or Claim 2 wherein the LHRH antagonist is a compound of the Formula:

$$\underline{a}-\underline{b}-\underline{c}-\underline{d}-\underline{e}-\underline{f}-\underline{g}-\underline{h}-Pro-\underline{j} \quad (I)$$
1 2 3 4 5 6 7 8 9 10

or a pharmaceutically acceptable salt thereof, wherein:
a is an amino acyl residue selected from the group consisting of either the D- or the L- isomer of:
N-Ac-D,L-$\Delta^{3,4}$-prolyl,
N-Ac-D,L-prolyl,
N-Ac-D,L-alkylprolyl,
N-Ac-D,L-phenylalanyl,
N-Ac-D,L-p-chlorophenylalanyl,
N-Ac-D,L-seryl,
N-Ac-D,L-threonyl,
N-Ac-D,L-alanyl,
N-Ac-3-(1-naphthyl)-D,L-alanyl,
N-Ac-3-(2-naphthyl)-D,L-alanyl,
N-Ac-D,L-p-fluorophenylalanyl,
N-Ac-3-(2,4,6-trimethylphenyl)-D,L-alanyl, and
N-Ac-3-(4-trifluoromethylphenyl)-D,L-alanyl;
b is an amino acyl residue selected from the group consisting of:
D-phenylalanyl,
D-p-chlorophenylalanyl,
D-p-bromophenylalanyl,
D-p-fluorophenylalanyl,
D-p-nitrophenylalanyl,
2,2-diphenylglycyl,
3-(3,4,5-trimethoxyphenyl)-D-alanyl,
3-(2,4,6-trimethoxyphenyl)-D-alanyl,
D-α-methyl-p-chlorophenylalanyl, and
3-(2-naphthyl)-D-alanyl;
c is an amino acyl resdidue selected from the group consisting of:
D-tryptophanyl,
D-phenylalanyl,
D-pentamethylphenylalanyl,
3-(3-pyridyl)-D-alanyl,
3-(1-naphthyl)-D-alanyl, and
3-(2-naphthyl)-D-alanyl;
d is an amino acyl residue selected from the group consisting of:
L-seryl, and
D-alanyl;
e is an amino acyl residue selected from the group consisting of:
L-phenylalanyl,
L-tyrosyl,
3-(3-pyridyl)-alanyl,
arginyl,
or h;
f is an amino acyl residue selected from the group consisting of:
3-(2-naphthyl)-D-alanyl,
3-(3-pyridyl)-D-alanyl;
D-tyrosyl,
D-tryptophanyl,
D-nicotinyl-lysyl,
pyridylacetyl-lysyl,
D-Glu(AA),
orh;
g is an amino acyl residue selected from the group consisting of:
L-leucyl,
L-norleucyl,

L-phenylalanyl,
L-tryptophanyl,
L-norvalyl and
3-(2-naphthyl)-L-alanyl;
h is an amino acyl residue selected from the group consisting of the radicals represented by the following structural formulas:

(a)

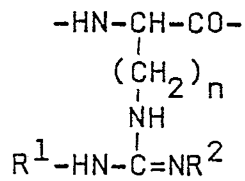

(II)

wherein
n is 1 to 5;
$R^1$ is alkyl of 1 to 6 carbon atoms or fluoroalkyl;
$R^2$ is hydrogen or $R^1$; or $R^1$-HN-C=$NR^2$ is a ring represented by the following structural formulas:

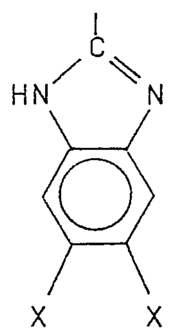

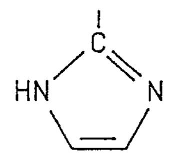

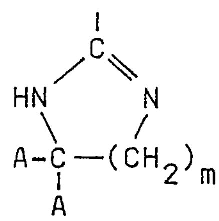

wherein m is 1 to 4; A is hydrogen or alkyl of 1 to 6 carbon atoms; and X is halo or A; and

(b)

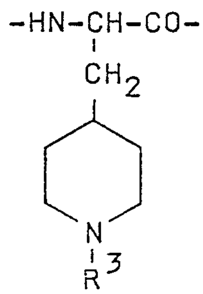

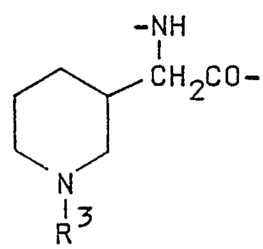

wherein $R^3$ is hydrogen, alkyl of 1 to 6 carbon atoms, phenyl or phenylloweralkyl; and
j is D-alaninamide; D-leucinamide; glycinamide; or -$NHR^4$ wherein $R^4$ is lower alkyl or $NHCONH_2$.

4. The composition of Claim 1, 2 or 3 wherein the 19-nor progestational agent is selected from the group consisting of norethindrone, norethindrone acetate, ethynodiol diacetate, norethynodrel, norgestrel and lynestrenol.

5. The composition of Claim 4 wherein the progestational agent is norethindrone.

6. The composition of Claim 5 wherein the antagonist is:
N-Ac-D-Nal(2)-D-pCl-Phe-D-Pal(3)-Ser-Tyr-D-Deh-Leu-Deh-Pro-D-Ala$NH_2$;
or an optical isomer thereof; or a pharmaceutically acceptable salt thereof.

7. The composition of Claim 5 wherein the antagonist is:
N-Ac-D-Nal(2)-D-pCl-Phe-D-Pal(3)-Ser-Tyr-D-Pal(3)-Leu-Deh-Pro-D-Ala$NH_2$;
or an optical isomer thereof; or a pharmaceutically acceptable salt thereof.

8. A composition according to Claim 1 for inhibiting ovulation, treating endometriosis, treating breast cancer, treating uterine leiomyomata, treating polycystic ovarian disease, or treating precocious puberty,

in a female mammalian subject.

9. An LHRH antagonist in combination with a menopausal-symptom-alleviating amount of a 19-nor progestational agent for pharmaceutical use.

10. The combination of Claim 9 for inhibiting ovulation, treating endometriosis, treating breast cancer, treating uterine leiomyomata, treating polycystic ovarian disease, or treating precocious puberty in a female mammalian subject.

11. The use of an LHRH antagonist and a menopausal-symptom-alleviating amount of a 19-nor progestational agent for preparing a pharmaceutical composition.

12. The use according to Claim 11 wherein the composition is for inhibiting ovulation, treating endometriosis, treating breast cancer, treating uterine leiomyomata, treating polycystic ovarian disease, or treating precocious puberty in a female mammalian subject.

13. A pharmaceutical package comprising an LHRH antagonist in a dosage form together with instructions prescribing co-administration of a menopausal-symptom-alleviating amount of a 19-nor progestational agent.

14. A pharmaceutical package comprising a 19-nor progestational agent in a menopausal-symptom-alleviating dosage form together with instructions prescribing co-administration of an LHRH antagonist.

15. A pharmaceutical package comprising an LHRH antagonist in a dosage form presented in said package in combination with a 19-nor progestational agent in a menopausal-symptom-alleviating dosage form.

**Claims for the following Contracting States: ES,GR**

1. A process comprising the preparation of a pharmaceutical composition, the pharmaceutical composition comprising an LHRH antagonist and a menopausal-symptom alleviating amount of a 19-nor progestational agent arranged for simultaneous or sequential administration.

2. A process according to Claim 1, wherein the LHRH antagonist and the 19-nor progestational agent are in a single formulation.

3. A process according to Claim 1 or Claim 2 wherein the LHRH antagonist is a compound of the Formula:

$$\underset{1}{\underline{a}}-\underset{2}{\underline{b}}-\underset{3}{\underline{c}}-\underset{4}{\underline{d}}-\underset{5}{\underline{e}}-\underset{6}{\underline{f}}-\underset{7}{\underline{g}}-\underset{8}{\underline{h}}-\underset{9}{\mathrm{Pro}}-\underset{10}{\underline{j}} \qquad (I)$$

or a pharmaceutically acceptable salt thereof, wherein:
a is an amino acyl residue selected from the group consisting of either the D- or the L- isomer of:
N-Ac-D,L-$\Delta^{3,4}$-prolyl,
N-Ac-D,L-prolyl,
N-Ac-D,L-alkylprolyl,
N-Ac-D,L-phenylalanyl,
N-Ac-D,L-p-chlorophenylalanyl,
N-Ac-D,L-seryl,
N-Ac-D,L-threonyl,
N-Ac-D,L-alanyl,
N-Ac-3-(1-naphthyl)-D,L-alanyl,
N-Ac-3-(2-naphthyl)-D,L-alanyl,
N-Ac-D,L-p-fluorophenylalanyl,
N-Ac-3-(2,4,6-trimethylphenyl)-D,L-alanyl, and
N-Ac-3-(4-trifluoromethylphenyl)-D,L-alanyl;
b is an amino acyl residue selected from the group consisting of:
D-phenylalanyl,
D-p-chlorophenylalanyl,
D-p-bromophenylalanyl,
D-p-fluorophenylalanyl,
D-p-nitrophenylalanyl,
2,2-diphenylglycyl,
3-(3,4,5-trimethoxyphenyl)-D-alanyl,
3-(2,4,6-trimethoxyphenyl)-D-alanyl,
D-α-methyl-p-chlorophenylalanyl, and
3-(2-naphthyl)-D-alanyl;
c is an amino acyl resdidue selected from the group consisting of:
D-tryptophanyl,
D-phenylalanyl,

D-pentamethylphenylalanyl,
3-(3-pyridyl)-D-alanyl,
3-(1-naphthyl)-D-alanyl, and
3-(2-naphthyl)-D-alanyl;
d is an amino acyl residue selected from the group consisting of:
L-seryl, and
D-alanyl;
e is an amino acyl residue selected from the group consisting of:
L-phenylalanyl,
L-tyrosyl,
3-(3-pyridyl)-alanyl,
arginyl,
or h;
f is an amino acyl residue selected from the group consisting of:
3-(2-naphthyl)-D-alanyl,
3-(3-pyridyl)-D-alanyl,
D-tyrosyl,
D-tryptophanyl,
D-nicotinyl-lysyl,
pyridylacetyl-lysyl,
D-Glu(AA),
orh;
g is an amino acyl residue selected from the group consisting of:
L-leucyl,
L-norleucyl,
L-phenylalanyl,
L-tryptophanyl,
L-norvalyl and
3-(2-naphthyl)-L-alanyl;
h is an amino acyl residue selected from the group consisting of the radicals represented by the following structural formulas:

(a)

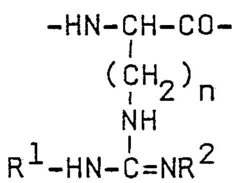

(II)

wherein
n is 1 to 5;
$R^1$ is alkyl of 1 to 6 carbon atoms or fluoroalkyl;
$R^2$ is hydrogen or $R^1$; or $R^1$-HN-C$=$$NR^2$ is a ring represented by the following structural formulas:

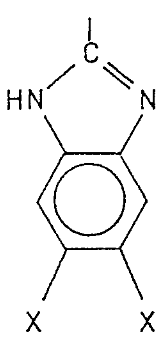

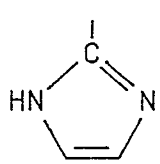

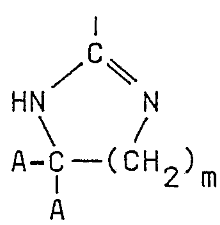

wherein m is 1 to 4; A is hydrogen or alkyl of 1 to 6 carbon atoms; and X is halo or A; and

(b)

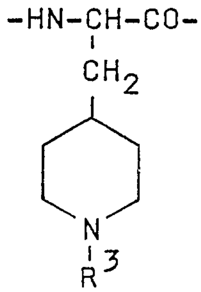

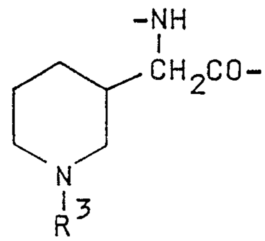

wherein $R^3$ is hydrogen, alkyl of 1 to 6 carbon atoms, phenyl or phenylloweralkyl; and
j is D-alaninamide; D-leucinamide; glycinamide; or $-NHR^4$ wherein $R^4$ is lower alkyl or $NHCONH_2$.

4. A process according to anyone of Claims 1, 2 or 3 wherein the 19-nor progestational agent is selected from the group consisting of norethindrone, norethindrone acetate, ethynodiol diacetate, norethynodrel, norgestrel and lynestrenol.

5. A process according to Claim 4 wherein the progestational agent is norethindrone.

6. A process according to Claim 5 wherein the antagonist is:
N-Ac-D-Nal(2)-D-pCl-Phe-D-Pal(3)-Ser-Tyr-D-Deh-Leu-Deh-Pro-D-Ala$NH_2$;
or an optical isomer thereof; or a pharmaceutically acceptable salt thereof.

7. A process according to Claim 5 wherein the antagonist is:
N-Ac-D-Nal(2)-D-pCl-Phe-D-Pal(3)-Ser-Tyr-D-Pal(3)-Leu-Deh-Pro-D-Ala$NH_2$;
or an optical isomer thereof; or a pharmaceutically acceptable salt thereof.

8. A process according to Claim 1 comprising the preparation of a pharmaceutical composition for inhibiting ovulation, treating endometriosis, treating breast cancer, treating uterine leiomyomata, treating polycystic ovarian disease, or treating precocious puberty, in a female mammalian subject.

9. A process according to Claim 1 comprising the preparation of a pharmaceutical composition, the pharmaceutical composition comprising an LHRH antagonist in combination with a menopausal-symptom-alleviating amount of a 19-nor progestational agent for pharmaceutical use.

10. The process of Claim 9 for preparation of a composition for inhibiting ovulation, treating endometriosis, treating breast cancer, treating uterine leiomyomata, treating polycystic ovarian disease, or treating precocious puberty in a female mammalian subject.

11. The use of an LHRH antagonist and a 19-nor progestational agent for preparing a pharmaceutical composition.

12. The use according to Claim 11 wherein the composition is for inhibiting ovulation, treating endometriosis, treating breast cancer, treating uterine leiomyomata, treating polycystic ovarian disease, or treating precocious puberty in a female mammalian subject.

13. A pharmaceutical package comprising an LHRH antagonist in a dosage form together with instructions prescribing co-administration of a menopausal-symptom-alleviating amount of a 19-nor progestational agent.

14. A pharmaceutical package comprising a 19-nor progestational agent in a menopausal-symptom-alleviating dosage form together with instructions prescribing co-administration of an LHRH antagonist.

15. A pharmaceutical package comprising an LHRH antagonist in a dosage form presented in said package in combination with a 19-nor progestational agent in a menopausal-symptom-alleviating dosage form.